# EUROPEAN PATENT APPLICATION

(11) **EP 3 725 896 A1**
(43) Date of publication of application: **21.10.2020**
(21) Application number: 18887319.4
(22) Date of filing: 13.12.2018
(51) Int. Cl.: C12Q 1/6886, C12N 15/09, C12Q 1/6869

(54) **METHOD FOR ASSISTING IN DETECTION OF BREAST CANCER**

(30) Priority: 13.12.2017 JP 2017238811
(71) Applicant: Hiroshima University, Higashihiroshima-shi Hiroshima 739-8511 (JP)
(72) Inventor: TAHARA, Hidetoshi, Hiroshima-shi, Hiroshima 734-8553 (JP); OCHIYA, Takahiro, Tokyo 104-0053 (JP)
(74) Representative: Mewburn Ellis LLP
(86) International application number: PCT/JP2018/045936
(87) International publication number: WO 2019/117257

(57) **Abstract**

Disclosed is a method of assisting the detection of breast cancer, assisting in highly accurate detection of breast cancer. In the method of assisting the detection of breast cancer, the abundance of at least one of miRNAs, isomiRs, precursor miRNAs, or transfer RNA fragments contained in a test sample isolated from a living body and having a specific nucleotide sequence is used as an index. A higher abundance of at least one of the miRNAs or the like whose nucleotide sequence is represented by any one of, for example, SEQ ID NOs: 1 to 19, 27, 28, and 34 to 51 than that of healthy subjects or a lower abundance of at least one of the miRNAs or the like whose nucleotide sequence is represented by any one of, for example, SEQ ID NOs: 20 to 26, 29 to 33, and 52 to 54 than that of healthy subjects indicates a higher likelihood of having breast cancer.

SEQUENCE LISTING

<110>  National University Corporation Hiroshima University

<120>  Method of aiding detection of breast cancer

<130>  PF631-PCT

<160>  282

<170>  PatentIn version 3.5

<210>  1
<211>  22
<212>  RNA
<213>  Homo sapiens

<400>  1
ggaagacuag ugauuuuguu gu                                              22


<210>  2
<211>  21
<212>  RNA
<213>  Homo sapiens

<400>  2
agcagcacau aaugguuugu g                                               21


<210>  3
<211>  22
<212>  RNA
<213>  Homo sapiens

<400>  3
acauucaacg cugucgguga gu                                              22


<210>  4
<211>  21
<212>  RNA
<213>  Homo sapiens

<400>  4
cauucaacgc ugucggugag u                                               21


<210>  5
<211>  20
<212>  RNA
<213>  Homo sapiens

<400>  5
auucaacgcu gucggugagu                                                 20


<210>  6
<211>  19
<212>  RNA
<213>  Homo sapiens

<400>  6

uucaacgcug ucggugagu 19

<210> 7
<211> 18
<212> RNA
<213> Homo sapiens

<400> 7
ucaacgcugu cggugagu 18

<210> 8
<211> 17
<212> RNA
<213> Homo sapiens

<400> 8
caacgcuguc ggugagu 17

<210> 9
<211> 16
<212> RNA
<213> Homo sapiens

<400> 9
aacgcugucg gugagu 16

<210> 10
<211> 15
<212> RNA
<213> Homo sapiens

<400> 10
acgcugucgg ugagu 15

<210> 11
<211> 15
<212> RNA
<213> Homo sapiens

<400> 11
aacgcugucg gugag 15

<210> 12
<211> 32
<212> RNA
<213> Homo sapiens

<400> 12
gcauuggugg uucaguggua gaauucucgc cu 32

<210> 13
<211> 31
<212> RNA
<213> Homo sapiens

<400> 13

gcauuggugg uucaguggua gaauucucgc c                                    31

<210>  14
<211>  32
<212>  RNA
<213>  Homo sapiens

<400>  14
gcgccgcugg uguaguggua ucaugcaaga uu                                   32

<210>  15
<211>  31
<212>  RNA
<213>  Homo sapiens

<400>  15
gcgccgcugg uguaguggua ucaugcaaga u                                    31

<210>  16
<211>  23
<212>  RNA
<213>  Homo sapiens

<400>  16
agcucggucu gaggccccuc agu                                             23

<210>  17
<211>  31
<212>  RNA
<213>  Homo sapiens

<400>  17
agcagagugg cgcagcggaa gcgugcuggg c                                    31

<210>  18
<211>  17
<212>  RNA
<213>  Homo sapiens

<400>  18
accccacucc ugguacc                                                    17

<210>  19
<211>  21
<212>  RNA
<213>  Homo sapiens

<400>  19
ucucccaacc cuuguaccag u                                               21

<210>  20
<211>  19
<212>  RNA
<213>  Homo sapiens

<400>  20

uagcagcacg uaaauauug                                                    19

<210>  21
<211>  21
<212>  RNA
<213>  Homo sapiens

<400>  21
gagguaguag uuuguacagu u                                                21

<210>  22
<211>  20
<212>  RNA
<213>  Homo sapiens

<400>  22
agguaguagu uuguacaguu                                                  20

<210>  23
<211>  19
<212>  RNA
<213>  Homo sapiens

<400>  23
gguaguaguu uguacaguu                                                   19

<210>  24
<211>  18
<212>  RNA
<213>  Homo sapiens

<400>  24
guaguaguuu guacaguu                                                    18

<210>  25
<211>  17
<212>  RNA
<213>  Homo sapiens

<400>  25
uaguaguuug uacaguu                                                     17

<210>  26
<211>  15
<212>  RNA
<213>  Homo sapiens

<400>  26
guaguuugua caguu                                                       15

<210>  27
<211>  32
<212>  RNA
<213>  Homo sapiens

<400>  27

gcgccgcugg uguaguggua ucaugcaaga uu                                    32

<210>  28
<211>  31
<212>  RNA
<213>  Homo sapiens

<400>  28
gcgccgcugg uguaguggua ucaugcaaga u                                     31

<210>  29
<211>  21
<212>  RNA
<213>  Homo sapiens

<400>  29
uacaguacug ugauaacuga a                                               21

<210>  30
<211>  20
<212>  RNA
<213>  Homo sapiens

<400>  30
gcucaguuca gcaggaacag                                                 20

<210>  31
<211>  16
<212>  RNA
<213>  Homo sapiens

<400>  31
aguucagcag gaacag                                                     16

<210>  32
<211>  22
<212>  RNA
<213>  Homo sapiens

<400>  32
uuggcacuag cacauuuuug cu                                              22

<210>  33
<211>  21
<212>  RNA
<213>  Homo sapiens

<400>  33
uggcacuagc acauuuuugc u                                               21

<210>  34
<211>  35
<212>  RNA
<213>  Homo sapiens

<400>  34

ucccuggugg ucuagugguu aggauucggc gcucu 35

<210> 35
<211> 32
<212> RNA
<213> Homo sapiens

<400> 35
ucccuggugg ucuagugguu aggauucggc gc 32

<210> 36
<211> 30
<212> RNA
<213> Homo sapiens

<400> 36
ucccuggugg ucuagugguu aggauucggc 30

<210> 37
<211> 32
<212> RNA
<213> Homo sapiens

<400> 37
gcaugggugg uucaguggua gaauucucgc cu 32

<210> 38
<211> 33
<212> RNA
<213> Homo sapiens

<400> 38
guuuccguag uguagugguu aucacguucg ccu 33

<210> 39
<211> 32
<212> RNA
<213> Homo sapiens

<400> 39
guuuccguag uguagugguu aucacguucg cc 32

<210> 40
<211> 32
<212> RNA
<213> Homo sapiens

<400> 40
gcgccgcugg uguaguggua ucaugcaaga uu 32

<210> 41
<211> 31
<212> RNA
<213> Homo sapiens

<400> 41

gcgccgcugg uguaguggua ucaugcaaga u                                    31

<210>  42
<211>  23
<212>  RNA
<213>  Homo sapiens

<400>  42
guccaguuuu cccaggaauc ccu                                            23

<210>  43
<211>  33
<212>  RNA
<213>  Homo sapiens

<400>  43
gguuccauag uguagugguu aucacgucug cuu                                 33

<210>  44
<211>  32
<212>  RNA
<213>  Homo sapiens

<400>  44
gcccggcuag cucagucggu agagcaugag ac                                  32

<210>  45
<211>  33
<212>  RNA
<213>  Homo sapiens

<400>  45
guuuccguag uguagcgguu aucacauucg ccu                                 33

<210>  46
<211>  24
<212>  RNA
<213>  Homo sapiens

<400>  46
uagcuuauca gacugauguu gacu                                           24

<210>  47
<211>  33
<212>  RNA
<213>  Homo sapiens

<400>  47
gcccggcuag cucagucggu agagcauggg acu                                 33

<210>  48
<211>  32
<212>  RNA
<213>  Homo sapiens

<400>  48

gcauuggugg uucaguggua gaauucucgc cu                                    32

<210>  49
<211>  31
<212>  RNA
<213>  Homo sapiens

<400>  49
gcauuggugg uucaguggua gaauucucgc c                                     31

<210>  50
<211>  32
<212>  RNA
<213>  Homo sapiens

<400>  50
ggggauguag cucaguggua gagcgcaugc uu                                    32

<210>  51
<211>  33
<212>  RNA
<213>  Homo sapiens

<400>  51
gcccggcuag cucagucggu agagcauggg acu                                   33

<210>  52
<211>  32
<212>  RNA
<213>  Homo sapiens

<400>  52
gcaugggugg uucaguggua gaauucucgc cu                                    32

<210>  53
<211>  21
<212>  RNA
<213>  Homo sapiens

<400>  53
uacccuguag auccgaauuu g                                                21

<210>  54
<211>  19
<212>  RNA
<213>  Homo sapiens

<400>  54
aagcugccag uugaagaac                                                   19

<210>  55
<211>  22
<212>  RNA
<213>  Homo sapiens

<400>  55

ccaauauuac ugugcugcuu ua                                                22

<210>  56
<211>  20
<212>  RNA
<213>  Homo sapiens

<400>  56
ugagguagua gguuguauag                                                    20

<210>  57
<211>  20
<212>  RNA
<213>  Homo sapiens

<400>  57
ugagguagua gguugugugg                                                    20

<210>  58
<211>  22
<212>  RNA
<213>  Homo sapiens

<400>  58
ugagguagua gguugugugg uu                                                 22

<210>  59
<211>  21
<212>  RNA
<213>  Homo sapiens

<400>  59
ugagguagua gguugugugg u                                                  21

<210>  60
<211>  20
<212>  RNA
<213>  Homo sapiens

<400>  60
ugagguagua gauuguauag                                                    20

<210>  61
<211>  21
<212>  RNA
<213>  Homo sapiens

<400>  61
ugagguagua guuuguacag u                                                  21

<210>  62
<211>  20
<212>  RNA
<213>  Homo sapiens

<400>  62

ugagguagua guuuguacag                                                    20


<210>  63
<211>  21
<212>  RNA
<213>  Homo sapiens

<400>  63
ugagguagua guuugugcug u                                                  21


<210>  64
<211>  20
<212>  RNA
<213>  Homo sapiens

<400>  64
ugagguagua guuugugcug                                                    20


<210>  65
<211>  21
<212>  RNA
<213>  Homo sapiens

<400>  65
guacaguacu gugauaacug a                                                  21


<210>  66
<211>  22
<212>  RNA
<213>  Homo sapiens

<400>  66
uacaguacug ugauaacuga ag                                                 22


<210>  67
<211>  20
<212>  RNA
<213>  Homo sapiens

<400>  67
uacaguacug ugauaacuga                                                    20


<210>  68
<211>  20
<212>  RNA
<213>  Homo sapiens

<400>  68
agcagcauug uacagggcua                                                    20


<210>  69
<211>  19
<212>  RNA
<213>  Homo sapiens

<400>  69

agcagcauug uacagggcu                                                    19


<210>  70
<211>  21
<212>  RNA
<213>  Homo sapiens

<400>  70
agcagcauug uacagggcua u                                                 21


<210>  71
<211>  23
<212>  RNA
<213>  Homo sapiens

<400>  71
aaaagugcuu acagugcagg uag                                               23


<210>  72
<211>  20
<212>  RNA
<213>  Homo sapiens

<400>  72
uaaagugcug acagugcaga                                                   20


<210>  73
<211>  21
<212>  RNA
<213>  Homo sapiens

<400>  73
uaaagugcug acagugcaga u                                                 21


<210>  74
<211>  22
<212>  RNA
<213>  Homo sapiens

<400>  74
cagugcaaug uuaaaagggc au                                                22


<210>  75
<211>  21
<212>  RNA
<213>  Homo sapiens

<400>  75
cagugcaaug uuaaaagggc a                                                 21


<210>  76
<211>  22
<212>  RNA
<213>  Homo sapiens

<400>  76

accacagggu agaaccacgg ac                                                22

<210>  77
<211>  23
<212>  RNA
<213>  Homo sapiens

<400>  77
accacagggu agaaccacgg aca                                               23

<210>  78
<211>  20
<212>  RNA
<213>  Homo sapiens

<400>  78
cccauaaagu agaaagcacu                                                   20

<210>  79
<211>  19
<212>  RNA
<213>  Homo sapiens

<400>  79
uacaguauag augauguac                                                    19

<210>  80
<211>  22
<212>  RNA
<213>  Homo sapiens

<400>  80
guccaguuuu cccaggaauc cc                                                22

<210>  81
<211>  21
<212>  RNA
<213>  Homo sapiens

<400>  81
ugagaacuga auuccauggg u                                                 21

<210>  82
<211>  22
<212>  RNA
<213>  Homo sapiens

<400>  82
ugagaacuga auuccauggg uu                                                22

<210>  83
<211>  22
<212>  RNA
<213>  Homo sapiens

<400>  83

ucucccaacc cuuguaccag ug                                                22

<210>  84
<211>  21
<212>  RNA
<213>  Homo sapiens

<400>  84
ucgaggagcu cacagucuag u                                                 21

<210>  85
<211>  21
<212>  RNA
<213>  Homo sapiens

<400>  85
uagcagcaca uaaugguuug u                                                 21

<210>  86
<211>  20
<212>  RNA
<213>  Homo sapiens

<400>  86
uagcagcaca uaaugguuug                                                   20

<210>  87
<211>  20
<212>  RNA
<213>  Homo sapiens

<400>  87
uagcagcaca ucaugguuua                                                   20

<210>  88
<211>  19
<212>  RNA
<213>  Homo sapiens

<400>  88
uagcagcaca ucaugguuu                                                    19

<210>  89
<211>  23
<212>  RNA
<213>  Homo sapiens

<400>  89
uagcagcacg uaaauauugg cgu                                               23

<210>  90
<211>  20
<212>  RNA
<213>  Homo sapiens

<400>  90

uagcagcacg uaaauauugg                                                20

<210>  91
<211>  21
<212>  RNA
<213>  Homo sapiens

<400>  91
uagcagcacg uaaauauugg c                                              21

<210>  92
<211>  20
<212>  RNA
<213>  Homo sapiens

<400>  92
accaauauua cugugcugcu                                                20

<210>  93
<211>  20
<212>  RNA
<213>  Homo sapiens

<400>  93
caaagugcuu acagugcagg                                                20

<210>  94
<211>  21
<212>  RNA
<213>  Homo sapiens

<400>  94
caaagugcuu acagugcagg u                                              21

<210>  95
<211>  22
<212>  RNA
<213>  Homo sapiens

<400>  95
aaagugcuua cagugcaggu ag                                             22

<210>  96
<211>  23
<212>  RNA
<213>  Homo sapiens

<400>  96
aacauucaac gcugucggug agu                                            23

<210>  97
<211>  23
<212>  RNA
<213>  Homo sapiens

<400>  97

uaaggugcau cuagugcaga uag                                              23

<210>  98
<211>  22
<212>  RNA
<213>  Homo sapiens

<400>  98
uaaggugcau cuagugcaga ua                                               22

<210>  99
<211>  21
<212>  RNA
<213>  Homo sapiens

<400>  99
uaaggugcau cuagugcaga u                                                21

<210>  100
<211>  20
<212>  RNA
<213>  Homo sapiens

<400>  100
uaaggugcau cuagugcaga                                                  20

<210>  101
<211>  24
<212>  RNA
<213>  Homo sapiens

<400>  101
caacggaauc ccaaaagcag cugu                                             24

<210>  102
<211>  22
<212>  RNA
<213>  Homo sapiens

<400>  102
caacggaauc ccaaaagcag cu                                               22

<210>  103
<211>  22
<212>  RNA
<213>  Homo sapiens

<400>  103
ugggucuuug cgggcgagau ga                                               22

<210>  104
<211>  21
<212>  RNA
<213>  Homo sapiens

<400>  104

uucaccaccu ucuccaccca g 21

<210> 105
<211> 23
<212> RNA
<213> Homo sapiens

<400> 105
ugugcaaauc uaugcaaaac uga 23

<210> 106
<211> 22
<212> RNA
<213> Homo sapiens

<400> 106
ugugcaaauc uaugcaaaac ug 22

<210> 107
<211> 21
<212> RNA
<213> Homo sapiens

<400> 107
ugugcaaauc uaugcaaaac u 21

<210> 108
<211> 20
<212> RNA
<213> Homo sapiens

<400> 108
ugugcaaauc caugcaaaac 20

<210> 109
<211> 21
<212> RNA
<213> Homo sapiens

<400> 109
ugugcaaauc caugcaaaac u 21

<210> 110
<211> 23
<212> RNA
<213> Homo sapiens

<400> 110
uaaagugcuu auagugcagg uag 23

<210> 111
<211> 22
<212> RNA
<213> Homo sapiens

<400> 111

uaaagugcuu auagugcagg ua                                                22

<210>  112
<211>  21
<212>  RNA
<213>  Homo sapiens

<400>  112
uaaagugcuu auagugcagg u                                                 21

<210>  113
<211>  23
<212>  RNA
<213>  Homo sapiens

<400>  113
caaagugcuc auagugcagg uag                                               23

<210>  114
<211>  21
<212>  RNA
<213>  Homo sapiens

<400>  114
caaagugcuc auagugcagg u                                                 21

<210>  115
<211>  21
<212>  RNA
<213>  Homo sapiens

<400>  115
gucaguuugu caaauacccc a                                                 21

<210>  116
<211>  22
<212>  RNA
<213>  Homo sapiens

<400>  116
gucaguuugu caaauacccc aa                                                22

<210>  117
<211>  20
<212>  RNA
<213>  Homo sapiens

<400>  117
ugucaguuug ucaaauaccc                                                   20

<210>  118
<211>  21
<212>  RNA
<213>  Homo sapiens

<400>  118

ugucaguuug ucaaauaccc c 21

<210> 119
<211> 23
<212> RNA
<213> Homo sapiens

<400> 119
ugucaguuug ucaaauaccc caa 23

<210> 120
<211> 22
<212> RNA
<213> Homo sapiens

<400> 120
ugucaguuug ucaaauaccc ca 22

<210> 121
<211> 19
<212> RNA
<213> Homo sapiens

<400> 121
uggcucaguu cagcaggaa 19

<210> 122
<211> 22
<212> RNA
<213> Homo sapiens

<400> 122
uggcucaguu cagcaggaac ag 22

<210> 123
<211> 20
<212> RNA
<213> Homo sapiens

<400> 123
cauugcacuu gucucggucu 20

<210> 124
<211> 21
<212> RNA
<213> Homo sapiens

<400> 124
cauugcacuu gucucggucu g 21

<210> 125
<211> 22
<212> RNA
<213> Homo sapiens

<400> 125

uucaaguaau ccaggauagg cu                                              22

<210>  126
<211>  21
<212>  RNA
<213>  Homo sapiens

<400>  126
uucaaguaau ucaggauagg u                                               21

<210>  127
<211>  20
<212>  RNA
<213>  Homo sapiens

<400>  127
uucaaguaau ucaggauagg                                                 20

<210>  128
<211>  22
<212>  RNA
<213>  Homo sapiens

<400>  128
uagcaccauc ugaaaucggu ua                                              22

<210>  129
<211>  22
<212>  RNA
<213>  Homo sapiens

<400>  129
uagcaccauu ugaaaucggu ua                                              22

<210>  130
<211>  21
<212>  RNA
<213>  Homo sapiens

<400>  130
uagcaccauu ugaaaucggu u                                               21

<210>  131
<211>  20
<212>  RNA
<213>  Homo sapiens

<400>  131
uguaaacauc cccgacugga                                                 20

<210>  132
<211>  23
<212>  RNA
<213>  Homo sapiens

<400>  132

guaaacaucc uugacuggaa gcu                                                23

<210>  133
<211>  24
<212>  RNA
<213>  Homo sapiens

<400>  133
uguaaacauc cuugacugga agcu                                               24

<210>  134
<211>  22
<212>  RNA
<213>  Homo sapiens

<400>  134
aaagcugggu ugagagggcg aa                                                 22

<210>  135
<211>  22
<212>  RNA
<213>  Homo sapiens

<400>  135
ucucacacag aaaucgcacc cg                                                 22

<210>  136
<211>  23
<212>  RNA
<213>  Homo sapiens

<400>  136
ucucacacag aaaucgcacc cgu                                                23

<210>  137
<211>  20
<212>  RNA
<213>  Homo sapiens

<400>  137
gcagugucuu agcugguugu                                                    20

<210>  138
<211>  19
<212>  RNA
<213>  Homo sapiens

<400>  138
ugaggggcag agagcgaga                                                     19

<210>  139
<211>  23
<212>  RNA
<213>  Homo sapiens

<400>  139

ugaggggcag agagcgagac uuu                                              23


<210>  140
<211>  23
<212>  RNA
<213>  Homo sapiens

<400>  140
aaugacacga ucacucccgu uga                                              23


<210>  141
<211>  21
<212>  RNA
<213>  Homo sapiens

<400>  141
aaccguuacc auuacugagu u                                                21


<210>  142
<211>  20
<212>  RNA
<213>  Homo sapiens

<400>  142
aaaccguuac cauuacugag                                                  20


<210>  143
<211>  25
<212>  RNA
<213>  Homo sapiens

<400>  143
aaaccguuac cauuacugag uuuag                                            25


<210>  144
<211>  24
<212>  RNA
<213>  Homo sapiens

<400>  144
aaaccguuac cauuacugag uuua                                             24


<210>  145
<211>  17
<212>  RNA
<213>  Homo sapiens

<400>  145
aaaccguuac cauuacu                                                     17


<210>  146
<211>  23
<212>  RNA
<213>  Homo sapiens

<400>  146

aaaccguuac cauuacugag uuu                                              23

<210>  147
<211>  19
<212>  RNA
<213>  Homo sapiens

<400>  147
aaaccguuac cauuacuga                                                   19

<210>  148
<211>  21
<212>  RNA
<213>  Homo sapiens

<400>  148
aaaccguuac cauuacugag u                                                21

<210>  149
<211>  22
<212>  RNA
<213>  Homo sapiens

<400>  149
aaaccguuac cauuacugag uu                                               22

<210>  150
<211>  20
<212>  RNA
<213>  Homo sapiens

<400>  150
uccuguacug agcugccccg                                                  20

<210>  151
<211>  21
<212>  RNA
<213>  Homo sapiens

<400>  151
gaagacuagu gauuuuguug u                                                21

<210>  152
<211>  20
<212>  RNA
<213>  Homo sapiens

<400>  152
gaagacuagu gauuuuguug                                                  20

<210>  153
<211>  22
<212>  RNA
<213>  Homo sapiens

<400>  153

uauugcacuu gucccggccu gu                                                22

<210>  154
<211>  21
<212>  RNA
<213>  Homo sapiens

<400>  154
uauugcacuu gucccggccu g                                                 21

<210>  155
<211>  23
<212>  RNA
<213>  Homo sapiens

<400>  155
caaagugcug uucgugcagg uag                                               23

<210>  156
<211>  20
<212>  RNA
<213>  Homo sapiens

<400>  156
caaagugcug uucgugcagg                                                   20

<210>  157
<211>  21
<212>  RNA
<213>  Homo sapiens

<400>  157
caaagugcug uucgugcagg u                                                 21

<210>  158
<211>  30
<212>  RNA
<213>  Homo sapiens

<400>  158
ggggguguag cucaguggua gagcgcgugc                                       30

<210>  159
<211>  26
<212>  RNA
<213>  Homo sapiens

<400>  159
ucccuggugg ucuagugguu aggauu                                            26

<210>  160
<211>  31
<212>  RNA
<213>  Homo sapiens

<400>  160

cgccgcuggu guagugguau caugcaagau u                                    31

<210>  161
<211>  29
<212>  RNA
<213>  Homo sapiens

<400>  161
cgccgcuggu guagugguau caugcaaga                                        29

<210>  162
<211>  30
<212>  RNA
<213>  Homo sapiens

<400>  162
cgccgcuggu guagugguau caugcaagau                                       30

<210>  163
<211>  30
<212>  RNA
<213>  Homo sapiens

<400>  163
gcgccgcugg uguaguggua ucaugcaaga                                       30

<210>  164
<211>  26
<212>  RNA
<213>  Homo sapiens

<400>  164
gcgccgcugg uguaguggua ucaugc                                           26

<210>  165
<211>  22
<212>  RNA
<213>  Homo sapiens

<400>  165
gcgccgcugg uguaguggua uc                                               22

<210>  166
<211>  27
<212>  RNA
<213>  Homo sapiens

<400>  166
gcgccgcugg uguaguggua ucaugca                                          27

<210>  167
<211>  25
<212>  RNA
<213>  Homo sapiens

<400>  167

gcaugggugg uucaguggua gaauu                                          25

<210>  168
<211>  30
<212>  RNA
<213>  Homo sapiens

<400>  168
agcagagugg cgcagcggaa gcgugcuggg                                     30

<210>  169
<211>  29
<212>  RNA
<213>  Homo sapiens

<400>  169
agcagagugg cgcagcggaa gcgugcugg                                      29

<210>  170
<211>  31
<212>  RNA
<213>  Homo sapiens

<400>  170
gcccggcuag cucagucggu agagcauggg a                                   31

<210>  171
<211>  32
<212>  RNA
<213>  Homo sapiens

<400>  171
gcccggcuag cucagucggu agagcauggg ac                                  32

<210>  172
<211>  31
<212>  RNA
<213>  Homo sapiens

<400>  172
guuuccguag uguagugguu aucacguucg c                                   31

<210>  173
<211>  31
<212>  RNA
<213>  Homo sapiens

<400>  173
guuuccguag uguagcgguu aucacauucg c                                   31

<210>  174
<211>  30
<212>  RNA
<213>  Homo sapiens

<400>  174

gcauuggugg uucaguggua gaauucucgc                                    30


<210>  175
<211>  17
<212>  RNA
<213>  Homo sapiens

<400>  175
cacaugaaaa aaugcuc                                                  17


<210>  176
<211>  15
<212>  RNA
<213>  Homo sapiens

<400>  176
caugaaaaaa ugcuc                                                    15


<210>  177
<211>  17
<212>  RNA
<213>  Homo sapiens

<400>  177
gacucuuagc gguggau                                                  17


<210>  178
<211>  29
<212>  RNA
<213>  Homo sapiens

<400>  178
ccgcuggugu agugguauca ugcaagauu                                     29


<210>  179
<211>  23
<212>  RNA
<213>  Homo sapiens

<400>  179
ggagagaacg cggucugagu ggu                                           23


<210>  180
<211>  16
<212>  RNA
<213>  Homo sapiens

<400>  180
gacucuuagc ggugga                                                   16


<210>  181
<211>  28
<212>  RNA
<213>  Homo sapiens

<400>  181

gagagggaga gaacgcgguc ugaguggu                                          28

<210>  182
<211>  27
<212>  RNA
<213>  Homo sapiens

<400>  182
agagggagag aacgcggucu gaguggu                                           27

<210>  183
<211>  18
<212>  RNA
<213>  Homo sapiens

<400>  183
guccaguuuu cccaggaa                                                     18

<210>  184
<211>  19
<212>  RNA
<213>  Homo sapiens

<400>  184
ugucaguuug ucaaauacc                                                    19

<210>  185
<211>  17
<212>  RNA
<213>  Homo sapiens

<400>  185
guccaguuuu cccagga                                                      17

<210>  186
<211>  17
<212>  RNA
<213>  Homo sapiens

<400>  186
aucacauugc cagggau                                                      17

<210>  187
<211>  24
<212>  RNA
<213>  Homo sapiens

<400>  187
gguguagugg uaucaugcaa gauu                                              24

<210>  188
<211>  19
<212>  RNA
<213>  Homo sapiens

<400>  188

uggaguguga caauggugu                                                    19

<210>  189
<211>  18
<212>  RNA
<213>  Homo sapiens

<400>  189
uucacagugg cuaaguuc                                                     18

<210>  190
<211>  20
<212>  RNA
<213>  Homo sapiens

<400>  190
guccaguuuu cccaggaauc                                                   20

<210>  191
<211>  15
<212>  RNA
<213>  Homo sapiens

<400>  191
gacucuuagc ggugg                                                        15

<210>  192
<211>  21
<212>  RNA
<213>  Homo sapiens

<400>  192
aacccguaga uccgaucuug u                                                 21

<210>  193
<211>  22
<212>  RNA
<213>  Homo sapiens

<400>  193
uguaguguuu ccuacuuuau gg                                                22

<210>  194
<211>  27
<212>  RNA
<213>  Homo sapiens

<400>  194
gcugguguag ugguaucaug caagauu                                           27

<210>  195
<211>  19
<212>  RNA
<213>  Homo sapiens

<400>  195

guccaguuuu cccaggaau                                                    19


<210>  196
<211>  20
<212>  RNA
<213>  Homo sapiens

<400>  196
uggaguguga caaugguguu                                                   20


<210>  197
<211>  21
<212>  RNA
<213>  Homo sapiens

<400>  197
uguaaacauc cucgacugga a                                                 21


<210>  198
<211>  20
<212>  RNA
<213>  Homo sapiens

<400>  198
uucacagugg cuaaguucug                                                   20


<210>  199
<211>  22
<212>  RNA
<213>  Homo sapiens

<400>  199
aucacauugc cagggauuac ca                                                22


<210>  200
<211>  25
<212>  RNA
<213>  Homo sapiens

<400>  200
guagucgugg ccgagugguu aaggc                                             25


<210>  201
<211>  23
<212>  RNA
<213>  Homo sapiens

<400>  201
cccccacugc uaaauuugac ugg                                               23


<210>  202
<211>  18
<212>  RNA
<213>  Homo sapiens

<400>  202

guuuccguag uguagugg                                                    18


<210>  203
<211>  26
<212>  RNA
<213>  Homo sapiens

<400>  203
ucccacaugg ucuagcgguu aggauu                                          26


<210>  204
<211>  25
<212>  RNA
<213>  Homo sapiens

<400>  204
ggcucguugg ucuaggggua ugauu                                           25


<210>  205
<211>  19
<212>  RNA
<213>  Homo sapiens

<400>  205
aaccguuacc auuacugag                                                  19


<210>  206
<211>  24
<212>  RNA
<213>  Homo sapiens

<400>  206
ccccccacug cuaaauuuga cugg                                            24


<210>  207
<211>  22
<212>  RNA
<213>  Homo sapiens

<400>  207
aaaagugcuu acagugcagg ua                                              22


<210>  208
<211>  21
<212>  RNA
<213>  Homo sapiens

<400>  208
aauggcgcca cuagguugu g                                                21


<210>  209
<211>  32
<212>  RNA
<213>  Homo sapiens

<400>  209

guuuccguag uguagcgguu aucacauucg cc                                    32


<210>  210
<211>  23
<212>  RNA
<213>  Homo sapiens

<400>  210
ucccuggugg ucuagugguu agg                                              23


<210>  211
<211>  28
<212>  RNA
<213>  Homo sapiens

<400>  211
gcccggcuag cucagucggu agagcaug                                         28


<210>  212
<211>  22
<212>  RNA
<213>  Homo sapiens

<400>  212
agcagcauug uacagggcua ug                                               22


<210>  213
<211>  24
<212>  RNA
<213>  Homo sapiens

<400>  213
gcaugggugg uucaguggua gaau                                             24


<210>  214
<211>  30
<212>  RNA
<213>  Homo sapiens

<400>  214
gcccggcuag cucagucggu agagcaugag                                       30


<210>  215
<211>  23
<212>  RNA
<213>  Homo sapiens

<400>  215
uagugcaaua uugcuuauag ggu                                              23


<210>  216
<211>  18
<212>  RNA
<213>  Homo sapiens

<400>  216

uccuguacug agcugccc                                                    18


<210>  217
<211>  23
<212>  RNA
<213>  Homo sapiens

<400>  217
gcaugggugg uucaguggua gaa                                              23


<210>  218
<211>  21
<212>  RNA
<213>  Homo sapiens

<400>  218
ugucuuacuc ccucaggcac a                                                21


<210>  219
<211>  16
<212>  RNA
<213>  Homo sapiens

<400>  219
uccuguacug agcugc                                                      16


<210>  220
<211>  22
<212>  RNA
<213>  Homo sapiens

<400>  220
caaagugcug uucgugcagg ua                                               22


<210>  221
<211>  23
<212>  RNA
<213>  Homo sapiens

<400>  221
guuuccguag uguagcgguu auc                                              23


<210>  222
<211>  27
<212>  RNA
<213>  Homo sapiens

<400>  222
ggcucguugg ucuaggggua ugauucu                                          27


<210>  223
<211>  28
<212>  RNA
<213>  Homo sapiens

<400>  223

ucccacaugg ucuagcgguu aggauucc 28

<210> 224
<211> 25
<212> RNA
<213> Homo sapiens

<400> 224
gacgaggugg ccgagugguu aaggc 25

<210> 225
<211> 24
<212> RNA
<213> Homo sapiens

<400> 225
aaccguuacc auuacugagu uuag 24

<210> 226
<211> 24
<212> RNA
<213> Homo sapiens

<400> 226
gacgaggugg ccgagugguu aagg 24

<210> 227
<211> 22
<212> RNA
<213> Homo sapiens

<400> 227
uggaagacua gugauuuugu ug 22

<210> 228
<211> 32
<212> RNA
<213> Homo sapiens

<400> 228
ggggguauag cucaguggua gagcauuuga cu 32

<210> 229
<211> 23
<212> RNA
<213> Homo sapiens

<400> 229
guagucgugg ccgagugguu aag 23

<210> 230
<211> 34
<212> RNA
<213> Homo sapiens

<400> 230

gcccggauga uccucagugg ucuggggugc aggc                                    34

<210>  231
<211>  24
<212>  RNA
<213>  Homo sapiens

<400>  231
guagucgugg ccgagugguu aagg                                              24

<210>  232
<211>  22
<212>  RNA
<213>  Homo sapiens

<400>  232
caaagugcuc auagugcagg ua                                                22

<210>  233
<211>  23
<212>  RNA
<213>  Homo sapiens

<400>  233
ggcugguccg aagguaguga guu                                               23

<210>  234
<211>  22
<212>  RNA
<213>  Homo sapiens

<400>  234
uaccgcacug ugguacuug cu                                                 22

<210>  235
<211>  27
<212>  RNA
<213>  Homo sapiens

<400>  235
ucccuggugg ucuagugguu aggauuc                                           27

<210>  236
<211>  23
<212>  RNA
<213>  Homo sapiens

<400>  236
gguuccauag uguagugguu auc                                               23

<210>  237
<211>  24
<212>  RNA
<213>  Homo sapiens

<400>  237

ucccuggugg ucuagugguu agga 24

<210> 238
<211> 23
<212> RNA
<213> Homo sapiens

<400> 238
gggggauuag cucaaauggu aga 23

<210> 239
<211> 18
<212> RNA
<213> Homo sapiens

<400> 239
gcuaaauuug acuggcuu 18

<210> 240
<211> 29
<212> RNA
<213> Homo sapiens

<400> 240
ucccacaugg ucuagcgguu aggauuccu 29

<210> 241
<211> 23
<212> RNA
<213> Homo sapiens

<400> 241
gcuucuguag uguagugguu auc 23

<210> 242
<211> 29
<212> RNA
<213> Homo sapiens

<400> 242
gcccggauga uccucagugg ucuggggug 29

<210> 243
<211> 31
<212> RNA
<213> Homo sapiens

<400> 243
ggggguauag cucaguggua gagcauuuga c 31

<210> 244
<211> 21
<212> RNA
<213> Homo sapiens

<400> 244
   cgcauccccu agggcauugg u                                              21

<210>  245
<211>  25
<212>  RNA
<213>  Homo sapiens

<400>  245
gccgaaauag cucaguuggg agagc                                           25

<210>  246
<211>  23
<212>  RNA
<213>  Homo sapiens

<400>  246
gacgaggugg ccgagugguu aag                                             23

<210>  247
<211>  28
<212>  RNA
<213>  Homo sapiens

<400>  247
guuuccguag uguagugguu aucacguu                                        28

<210>  248
<211>  26
<212>  RNA
<213>  Homo sapiens

<400>  248
gcccggcuag cucagucggu agagca                                          26

<210>  249
<211>  25
<212>  RNA
<213>  Homo sapiens

<400>  249
gcccggcuag cucagucggu agagc                                           25

<210>  250
<211>  30
<212>  RNA
<213>  Homo sapiens

<400>  250
guuuccguag uguagugguu aucacguucg                                      30

<210>  251
<211>  23
<212>  RNA
<213>  Homo sapiens

<400>  251

guuuccguag uguagugguu auc                                              23


<210>  252
<211>  25
<212>  RNA
<213>  Homo sapiens

<400>  252
ucccuggugg ucuagugguu aggau                                            25


<210>  253
<211>  29
<212>  RNA
<213>  Homo sapiens

<400>  253
ucccuggugg ucuagugguu aggauucgg                                        29


<210>  254
<211>  25
<212>  RNA
<213>  Homo sapiens

<400>  254
gggggauuag cucaaauggu agagc                                            25


<210>  255
<211>  26
<212>  RNA
<213>  Homo sapiens

<400>  255
gguuccauag uguagugguu aucacg                                           26


<210>  256
<211>  31
<212>  RNA
<213>  Homo sapiens

<400>  256
gggguauagc ucagugguag agcauuugac u                                     31


<210>  257
<211>  24
<212>  RNA
<213>  Homo sapiens

<400>  257
gggggauuag cucaaauggu agag                                             24


<210>  258
<211>  24
<212>  RNA
<213>  Homo sapiens

<400>  258

gaccucgugg cgcaacggua gcgc                                          24

<210>  259
<211>  26
<212>  RNA
<213>  Homo sapiens

<400>  259
guuuccguag uguagugguu aucacg                                        26

<210>  260
<211>  24
<212>  RNA
<213>  Homo sapiens

<400>  260
guuuccguag uguagugguu auca                                          24

<210>  261
<211>  25
<212>  RNA
<213>  Homo sapiens

<400>  261
ggggaauuag cucaaauggu agagc                                         25

<210>  262
<211>  29
<212>  RNA
<213>  Homo sapiens

<400>  262
guuuccguag uguagugguu aucacguuc                                     29

<210>  263
<211>  24
<212>  RNA
<213>  Homo sapiens

<400>  263
gaccucgugg cgcaauggua gcgc                                          24

<210>  264
<211>  24
<212>  RNA
<213>  Homo sapiens

<400>  264
ggggauuagc ucaaauggua gagc                                          24

<210>  265
<211>  25
<212>  RNA
<213>  Homo sapiens

<400>  265

guuuccguag uguagugguu aucac                                          25

<210>  266
<211>  23
<212>  RNA
<213>  Homo sapiens

<400>  266
uagcuuauca gacugauguu gac                                            23


<210>  267
<211>  22
<212>  RNA
<213>  Homo sapiens

<400>  267
aucacauugc cagggauuuc ca                                             22


<210>  268
<211>  20
<212>  RNA
<213>  Homo sapiens

<400>  268
uucacagugg cuaaguuccg                                                20


<210>  269
<211>  28
<212>  RNA
<213>  Homo sapiens

<400>  269
ggcugguccg augguagugg guuaucag                                       28


<210>  270
<211>  22
<212>  RNA
<213>  Homo sapiens

<400>  270
uagcagcaca uaaugguuug ug                                             22


<210>  271
<211>  30
<212>  RNA
<213>  Homo sapiens

<400>  271
gcauuggugg uucaguggua gaauucucgc                                      30


<210>  272
<211>  22
<212>  RNA
<213>  Homo sapiens

<400>  272
uagcagcacg uaaauauugg cg                                             22

<210>  273
<211>  22
<212>  RNA
<213>  Homo sapiens

<400>  273
ugagguagua guuuguacag uu                                                22


<210>  274
<211>  23
<212>  RNA
<213>  Homo sapiens

<400>  274
uuuggcacua gcacauuuuu gcu                                               23


<210>  275
<211>  31
<212>  RNA
<213>  Homo sapiens

<400>  275
ucccuggugg ucuagugguu aggauucggc g                                      31


<210>  276
<211>  30
<212>  RNA
<213>  Homo sapiens

<400>  276
gcaugggugg uucaguggua gaauucucgc                                         30


<210>  277
<211>  31
<212>  RNA
<213>  Homo sapiens

<400>  277
guuuccguag uguagugguu aucacguucg c                                      31


<210>  278
<211>  22
<212>  RNA
<213>  Homo sapiens

<400>  278
guccaguuuu cccaggaauc cc                                                22


<210>  279
<211>  22
<212>  RNA
<213>  Homo sapiens

<400>  279
uagcuuauca gacugauguu ga                                                22

<210>  280
<211>  30
<212>  RNA
<213>  Homo sapiens

<400>  280
gcauuggugg uucaguggua gaauucucgc                                        30

<210>  281
<211>  22
<212>  RNA
<213>  Homo sapiens

<400>  281
aagcugccag uugaagaacu gu                                               22

<210>  282
<211>  23
<212>  RNA
<213>  Homo sapiens

<400>  282
caaagugcuu acagugcagg uag                                              23

## Description

### TECHNICAL FIELD

The present invention relates to a method of assisting the detection of breast cancer.

### BACKGROUND ART

Diagnostic imaging, such as ultrasound imaging or mammography, or palpation is routinely performed as a diagnostic test for breast cancer. However, it is reported that some cases of breast cancer are missed by those test methods, and stage 0 breast cancer preceding tumor mass formation is also not detectable at all by the test methods.

On the other hand, methods in which the abundance of microRNA (hereinafter referred to as "miRNA") in blood is used as an index to detect breast cancer have been proposed (Patent Documents 1 to 3).

### PRIOR ART DOCUMENTS

### Patent Documents

Patent Document 1: JP 2009-505639 A
Patent Document 2: JP 2014-117282 A
Patent Document 3: JP 2016-25853 A

### SUMMARY OF THE INVENTION

### PROBLEM TO BE SOLVED BY THE INVENTION

As described above, various miRNAs have been proposed as indexes for the detection of breast cancer and, needless to say, it is advantageous if breast cancer can be detected with higher accuracy.

Thus, an object of the present invention is to provide a method of assisting the detection of breast cancer which assists in highly accurate detection of breast cancer.

### MEANS FOR SOLVING THE PROBLEM

As a result of intensive study, the inventors newly found miRNAs, isoform miRNAs (isomiRs), transfer RNA fragments (tRFs), and non-coding RNA fragments (RRNAs, snoRNAs, LincRNAs) which increase or decrease in abundance in breast cancer, and discovered that use of these as indexes enables highly accurate detection of breast cancer, to thereby complete the present invention.

That is, the present invention provides the following:
(1) A method of assisting the detection of breast cancer, using as an index the abundance of at least one of miRNAs, isoform miRNAs (isomiRs), precursor miRNAs, transfer RNA fragments (tRFs), or non-coding RNA fragments (RRNAs, snoRNAs, or LincRNAs) contained in a test sample isolated from a living body, whose nucleotide sequence is represented by any one of SEQ ID NOs: 2, 21, 22, 23, 24, 26, 30, 31, 32, 33, 34, 152, 151, 15, 28, 41, 1, 14, 27, 40. 25. 12, 160, 3 to 11, 13, 16 to 20, 29, 35 to 39, 42 to 150, 153 to 159, and 161 to 269, wherein a higher abundance of at least one of the miRNAs, isomiRs, precursor miRNAs, transfer RNA fragments, or non-coding RNA fragments whose nucleotide sequence is represented by any one of SEQ ID NOs: 1 to 19, 27, 28, 34 to 51. 74, 76, 77, 80 to 84, 96, 101 to 104, 115 to 122, 125, 128, 134 to 139, 151, 152, 159 to 165, 168, 169, 174, and 175 to 199 than that of healthy subjects or a lower abundance of at least one of the miRNAs, isomiRs, precursor miRNAs, transfer RNA fragments, or non-coding RNA fragments whose nucleotide sequence is represented by any one of SEQ ID NOs: 20 to 26, 29 to 33, 52 to 54, 56 to 73, 75, 78 to 79, 85 to 95, 97 to 100, 105 to 114, 123, 124, 126, 127, 129 to 133, 140 to 150, 153 to 158, 166, 167, 170 to 173, and 200 to 269 than that of healthy subjects indicates a higher likelihood of having breast cancer.
(2) The method according to (1), wherein the abundance of at least one of miRNAs, isoform miRNAs (isomiRs), or transfer RNA fragments (tRFs) whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, 160, 3 to 11, 13, 16 to 20, 29, 35 to 39, 42 to 150, 153 to 159, and 161 to 174 is used as an index.
(3) The method according to (1), wherein the abundance of at least one of miRNAs, isomiRs, precursor miRNAs, transfer RNA fragments, or non-coding RNA fragments whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, 160, 3 to 11, 13, 16, 20, 27, 29, 37 to 39, 41, 43, 45, 47 to 52, 56, 60, 66, 82, 86, 90 to 92, 107, 111, 112, 126, 127, 130, 137, 158, 161, 162, 173, and 175 to 265 is used as an index.
(4) The method according to (3), wherein the abundance of at least one of miRNAs, isomiRs, precursor miRNAs, or transfer RNA fragments whose nucleotide sequence is represented by any one of SEQ ID NOs: 2, 21, 22, 23, 24, 26, 30, 31, 32, 33, 34, 152, 151, 15, 28, 41, 1, 14, 27, 40. 25, 12, 160, 3 to 11, 13, 16, 20, 27, 29, 37 to 39, 41, 43, 45, 47 to 52, 56, 60, 66, 82, 86, 90 to 92, 107, 111, 112, 126, 127, 130, 137, 158, 161, 162, and 173 is used as an index.
(5) The method according to (4), wherein the abundance of at least one of isomiRs or precursor miRNAs whose nucleotide sequence is represented by any one of SEQ ID NOs: 3 to 9 is used as an index.
(6) The method according to (2), wherein the abundance of at least one of miRNAs, isomiRs, precursor miRNAs, or transfer RNA fragments whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, and 160 is used as an index.
(7) The method according to (6), wherein the abundance of at least one of isomiRs or transfer RNA fragments whose nucleotide sequence is represented by SEQ ID NO: 152, 151, 15, 40, 41, 1, or 14 is used as an index.
(8) The method according to (2), wherein the abundance of at least one of isomiRs or transfer RNA fragments whose nucleotide sequence is represented by any one of SEQ ID NOs: 2, 21, 22, 23, 24, 26, 31 to 33, and 55 is used as an index.
(9) The method according to (2), comprising measuring the abundance ratio of isoforms (isomiRs) of miR-150-5p (SEQ ID NO: 83) and/or miR-26b-5p (SEQ ID NO: 126) to the same microRNA(s) in the mature miRNA form contained in serum or plasma isolated from a living body (where "the abundance of isoforms (isomiRs)" refers to the total abundance of sequences in which 1 to 5 nucleotides are deleted from or added to the 3' or 5' end of a mature miRNA), wherein a higher abundance ratio than that of healthy subjects indicates a higher likelihood of having breast cancer.
(10) The method according to (2), comprising measuring the abundance ratio of isoforms (isomiRs) of miR-93-5p (SEQ ID NO: 155) and/or miR-17-5p (SEQ ID NO: 282) to the same microRNA(s) in the mature miRNA form contained in serum or plasma isolated from a living body (where "the abundance of isoforms (isomiRs)" refers to the total abundance of sequences in which 1 to 5 nucleotides are deleted from or added to the 3' or 5' end of a mature miRNA), wherein a lower abundance ratio than that of healthy subjects indicates a higher likelihood of having breast cancer.

### EFFECT OF THE INVENTION

By the method of the present invention, breast cancer can be highly accurately and yet conveniently detected. Thus, the method of the present invention will greatly contribute to the detection of breast cancer.

### DETAILED DESCRIPTION OF THE INVENTION

As described above, the abundance of a particular molecule selected from miRNAs, isomiRs, precursor miRNAs, transfer RNA fragments, or non-coding RNA fragments (hereinafter sometimes referred to as "miRNAs or the like" for convenience) contained in a test sample isolated from a living body is used as an index in the method of the present invention. These miRNAs or the like themselves are known, and the nucleotide sequences thereof are as shown in Sequence Listing. The list of miRNAs or the like used in the method of the present invention is presented in Table 1.

**Table 1-1**

| SEQ ID NO: | Class | Archetype | Type | Length (nucleotides) | Sequence |
|---|---|---|---|---|---|
| I | isomiR | mir-7-1//mir-7-2//mir-7-3 5p | Mature 5' sub | 22 | ggaagacuagugauuuuguugu |
| 2 | isomiR | mir-15a 5p | Mature 5' sub | 21 | agcagcacauaaugguuugug |
| 3 | isomiR | mir-181a-2//mir-181a-1 5p | Mature 5' sub | 22 | acauucaacgcugucggugagu |
| 4 | isomiR | mir-181a-2//mir-181a-1 5p | Mature 5' sub | 21 | cauucaacgcugucggugagu |
| 5 | isomiR | mir-181a-2//mir-181a-1 5p | Mature 5' sub | 20 | auucaacgcugucggugagu |
| 6 | isomiR | mir-181a-2//mir-181a-1 5p | Mature 5' sub | 19 | uucaacgcugucggugagu |
| 7 | isomiR | mir-181a-2//mir-181a-1 5p | Mature 5' sub | 18 | ucaacgcugucggugagu |
| 8 | precursor | mir-181 a-2//mir-181a-1 5p | precursor miRNA | 17 | caacgcugucggugagu |
| 9 | precursor | mir-181a-2//mir-181a-1 5p | precursor miRNA | 16 | aacgcugucggugagu |
| 10 | precursor | mir-181a-2//mir-181a-1 5p | precursor miRNA | 15 | acgcugucggugagu |
| 11 | precursor | mir-181a-2//mir-181a-1 5p | precursormiRNA | 15 | aacgcugucggugag |
| 12 | tRF | Homo_sapiens_tRNA-Gly-CCC-1-1//···*1 //···*1 | Exact | 32 | gcauuggugguucagugguagaauucucgccu |
| 13 | tRF | Homo_ sapiens_tRNA-Gly-CCC-1-1//···*1 | Exact | 31 | gcauuggugguucagugguagaauucucgcc |
| 14 | tRF | Homo_sapiens_tRNA-Gly-CCC-2-1//Homo_sapi ens tRNA-Gly-CCC-2-2 | Exact | 32 | gcgccgcugguguagugguaucaugcaagauu |
| 15 | tRF | the same as above | Exact | 31 | gcgccgcugguguagugguaucaugcaagau |
| 16 | miRNA | mir-423 3p | Mature 3' | 23 | agcucggucugaggccccucagu |
| 17 | tRF | Homo_ sapiens_tRNA-iMet-CAT-1-1//···*2 | Exact | 31 | agcagaguggcgcagcggaagcgugcugggc |
| 18 | miRNA | mir-4286 5p | Mature 5' | 17 | accccacuccugguacc |
| 19 | isomiR | mir-150 5p | Mature 5' sub | 21 | ucucccaacccuuguaccagu |
| 20 | isomiR | mir-16-1//mir-16-2 5p | Mature 5' sub | 19 | uagcagcacguaaauauug |
| 21 | isomiR | let-7g 5p | Mature 5' sub | 21 | gagguaguaguuuguacaguu |
| 22 | isomiR | let-7g 5p | Mature 5' sub | 20 | agguaguaguuuguacaguu |
| 23 | isomiR | let-7g 5p | Mature 5' sub | 19 | gguaguaguuuguacaguu |
| 24 | isomiR | let-7g 5p | Mature 5' sub | 18 | guaguaguuuguacaguu |
| 25 | isomiR | let-7g 5p | Mature 5' sub | 17 | uaguaguuuguacaguu |
| 26 | precursor | let-7g 5p | precursor miRNA | 15 | guaguuuguacaguu |
| 27 | tRF | Homo_sapiens_tRNA-Gly-CCC-2-1//Homo_sapi ens_tRNA-Gly-CCC-2-2 | Exact | 32 | gcgccgcugguguagugguaucaugcaagauu |

**Table 1-2**

| SEQ ID NO: | Class | Archetype | Type | Length (nucleotides) | Sequence |
|---|---|---|---|---|---|
| 28 | tRF | the same as above | Exact | 31 | gcgccgcugguguagugguaucaugcaagau |
| 29 | miRNA | mir-101-1//mir-101-2 3p | Mature 3' | 21 | uacaguacugugauaacugaa |
| 30 | isomiR | mir-24-1//mir-24-2 3p | Mature 3' sub | 20 | gcucaguucagcaggaacag |
| 31 | precursor | mir-24-1//mir-24-2 3p | precursor miRNA | 16 | aguucagcaggaacag |
| 32 | isomiR | mir-96 5p | Mature 5' sub | 22 | uuggcacuagcacauuuuugcu |
| 33 | isomiR | mir-96 5p | Mature 5' sub | 21 | uggcacuagcacauuuuugcu |
| 34 | tRF | Homo_sapienstRNA-Gln-CTC-1-1//···*3 | Exact | 35 | ucccugguggucuagugguuaggauucggcgcucu |
| 35 | tRF | Homo_ sapiens_tRNA-Glu-CTC-1-1//···*3 | Exact | 32 | ucccugguggucuagugguuaggauucggcgc |
| 36 | tRF | Homo_sapiens_tRNA-Glu-CTC-1-1//···*3 | Exact | 30 | ucccugguggucuagugguuaggauucggc |
| 37 | tRF | Homo_ sapiens_tRNA-Gly-GCC-1-1//···*4 | Exact | 32 | gcaugggugguucagugguagaauucucgccu |
| 38 | tRF | Homo_sapiens_ tRNA-Val-AAC-1-1//···*5 | Exact | 33 | guuuccguaguguagugguuaucacguucgccu |
| 39 | tRF | Homo_sapiens_ tRNA-Val-AAC-1-1//···*5 | Exact | 32 | guuuccguaguguagugguuaucacguucgcc |
| 40 | tRF | Homo_sapiens_tRNA-Gly-CCC-2-1//Homo_sapi ens_tRNA-Gly-CCC-2-2 | Exact | 32 | gcgccgcugguguagugguaucaugcaagauu |
| 41 | tRF | the same as above | Exact | 31 | gcgccgcugguguagugguaucaugcaagau |
| 42 | miRNA | mir-145 5p | Mature 5' | 23 | guccaguuuucccaggaaucccu |
| 43 | tRF | Homo_sapiens_tRNA-Val-TAC-1-1//Homo_sapie ns_tRNA-Val-TAC-1-2 | Exact | 33 | gguuccauaguguagugguuaucacgucugcuu |
| 44 | tRF | Homo_ sapiens_tRNA-Lys-CTT-2-1//···*6 | Exact | 32 | gcccggcuagcucagucgguagagcaugagac |
| 45 | tRF | Homo_ sapiens_tRNA-Val-CAC-3-1 | Exact | 33 | guuuccguaguguagcgguuaucacauucgccu |
| 46 | isomiR | mir-21 5p | mature 5' super | 24 | uagcuuaucagacugauguugacu |
| 47 | tRF | Homo_sapiens_tRNA-Lys-CTT-1-1//···*7 | Exact | 33 | gcccggcuagcucagucgguagagcaugggacu |
| 48 | tRF | Homo_sapiens_tRNA-Gly-CCC-1-1//···*8 | Exact | 32 | gcauuggugguucagugguagaauucucgccu |
| 49 | tRF | Homo_ sapiens_tRNA-Gly-CCC-1-1//···*8 | Exact | 31 | gcauuggugguucagugguagaauucucgcc |
| 50 | tRF | Homo_ sapiens tRNA-Ala-AGC-4-1//···*9 | Exact | 32 | ggggauguagcucagugguagagcgcaugcuu |
| 51 | tRF | Homo_ sapiens_tRNA-Lys-CTT-1-1//···*10 | Exact | 33 | gcccggcuagcucagucgguagagcaugggacu |
| 52 | tRF | Homo_sapiens_tRNA-Gly-GCC-1-1//···* 11 | Exact | 32 | gcaugggugguucagugguagaauucucgccu |
| 53 | isomiR | mir-10a 5p | Mature 5' sub | 21 | uacccuguagauccgaauuug |
| 54 | isomiR | mir-22 3p | Mature 3' sub | 19 | aagcugccaguugaagaac |
| 55 | miRNA | mir-16-2 3p | Mature 3' | 22 | ccaauauuacugugcugcuuua |

**Table 1-3**

| SEQ ID NO: | Class | Archetype | Type | Length (nucleotides) | Sequence |
|---|---|---|---|---|---|
| 56 | isomiR | let-7a-1//let-7a-2//let-7a-3 5p | Mature 5' sub | 20 | ugagguaguagguuguauag |
| 57 | isomiR | let-7b 5p | Mature 5' sub | 20 | ugagguaguagguugugugg |
| 58 | miRNA | let-7b | Mature 5' | 22 | ugagguaguagguugugugguu |
| 59 | isomiR | let-7b 5p | Mature 5' sub | 21 | ugagguaguagguuguguggu |
| 60 | isomiR | let-7f-1//let-7f-2 5p | Mature 5' sub | 20 | ugagguaguagauuguauag |
| 61 | isomiR | let-7g 5p | Mature 5' sub | 21 | ugagguaguaguuuguacagu |
| 62 | isomiR | let-7g 5p | Mature 5' sub | 20 | ugagguaguaguuuguacag |
| 63 | isomiR | let-7i 5p | Mature 5' sub | 21 | ugagguaguaguuugugcugu |
| 64 | isomiR | let-7i 5p | Mature 5' sub | 20 | ugagguaguaguuugugcug |
| 65 | isomiR | mir-101-1//mir-101-2 3p | Mature 3' sub/super | 21 | guacaguacugugauaacuga |
| 66 | isomiR | mir-101-1//mir-101-2 3p | Mature 3' super | 22 | uacaguacugugauaacugaag |
| 67 | isomiR | mir-101-1//mir-101-2 3p | Mature 3' sub | 20 | uacaguacugugauaacuga |
| 68 | isomiR | mir-103a-2//mir-103a-1//mir-107 3p | Mature 3' sub | 20 | agcagcauuguacagggcua |
| 69 | isomiR | mir-103a-2//mir-103a-1//mir-107 3p | Mature 3' sub | 19 | agcagcauuguacagggcu |
| 70 | isomiR | mir-103a-2//mir-103a-1//mir-107 3p | Mature 3' sub | 21 | agcagcauuguacagggcuau |
| 71 | miRNA | mir-106a 5p | Mature 5' | 23 | aaaagugcuuacagugcagguag |
| 72 | isomiR | mir-106b 5p | Mature 5' sub | 20 | uaaagugcugacagugcaga |
| 73 | miRNA | mir-106b 5p | Mature 5' | 21 | uaaagugcugacagugcagau |
| 74 | miRNA | mir-130a 3p | Mature 3' | 22 | cagugcaauguuaaaagggcau |
| 75 | isomiR | mir-130a 3p | Mature 3' sub | 21 | cagugcaauguuaaaagggca |
| 76 | isomiR | mir-140 3p | Mature 3' sub/super | 22 | accacaggguagaaccacggac |
| 77 | isomiR | mir-140 3p | Mature 3' sub/super | 23 | accacaggguagaaecacggaca |
| 78 | isomiR | mir-142 5p | Mature 5' sub/super | 20 | cccauaaaguagaaagcacu |
| 79 | isomiR | mir-144 3p | Mature 3' sub | 19 | uacaguauagaugauguac |
| 80 | isomiR | mir-145 5p | Mature 5' sub | 22 | guccaguuuucccaggaauccc |
| 81 | isomiR | mir-146a 5p | Mature 5' sub | 21 | ugagaacugaauuccaugggu |

**Table 1-4**

| SEQ ID NO: | Class | Archetype | Type | Length (nucleotides) | Sequence |
|---|---|---|---|---|---|
| 82 | miRNA | mir-146a 5p | Mature 5' | 22 | ugagaacugaauuccauggguu |
| 83 | miRNA | mir-150 5p | Mature 5' | 22 | ucucccaacccuuguaccagug |
| 84 | miRNA | mir-151a 5p | Mature 5' | 21 | ucgaggagcucacagucuagu |
| 85 | isomiR | mir-15a 5p | Mature 5' sub | 21 | uagcagcacauaaugguuugu |
| 86 | isomiR | mir-15a 5p | Mature 5' sub | 20 | uagcagcacauaaugguuug |
| 87 | isomiR | mir-15b 5p | Mature 5' sub | 20 | uagcagcacaucaugguuua |
| 88 | isomiR | mir-15b 5p | Mature 5' sub | 19 | uagcagcacaucaugguuu |
| 89 | isomiR | mir-16-1//mir-16-2 5p | Mature 5' super | 23 | uagcagcacguaaauauuggcgu |
| 90 | isomiR | mir-16-1//mir-16-2 5p | Mature 5' sub | 20 | uagcagcacguaaauauugg |
| 91 | isomiR | mir-16-1//mir-16-2 5p | Mature 5' sub | 21 | uagcagcacguaaauauuggc |
| 92 | isomiR | mir-16-2 3p | Mature 3' sub/super | 20 | accaauauuacugugcugcu |
| 93 | isomiR | mir-17 5p | Mature 5' sub | 20 | caaagugcuuacagugcagg |
| 94 | isomiR | mir-17 5p | Mature 5' sub | 21 | caaagugcuuacagugcaggu |
| 95 | isomiR | mir-17//mir-106a 5p | Mature 5' sub | 22 | aaagugcuuacagugcagguag |
| 96 | miRNA | mir-181a-2//mir-181a-1 5p | Mature 5' | 23 | aacauucaacgcugucggugagu |
| 97 | miRNA | mir-18a 5p | Mature 5' | 23 | uaaggugcaucuagugcagauag |
| 98 | isomiR | mir-18a 5p | Mature 5' sub | 22 | uaaggugcaucuagugcagaua |
| 99 | isomiR | mir-18a 5p | Mature 5' sub | 21 | uaaggugcaucuagugcagau |
| 100 | isomiR | mir-18a 5p | Mature 5' sub | 20 | uaaggugcaucuagugcaga |
| 101 | isomiR | mir-191 5p | Mature 5' super | 24 | caacggaaucccaaaagcagcugu |
| 102 | isomiR | mir-191 5p | Mature 5' sub | 22 | caacggaaucccaaaagcagcu |
| 103 | miRNA | mir-193a 5p | Mature 5' | 22 | ugggucuuugcgggcgagauga |
| 104 | isomiR | mir-197 3p | Mature 3' sub | 21 | uucaccaccuucuccacccag |
| 105 | miRNA | mir-19a 3p | Mature 3' | 23 | ugugcaaaucuaugcaaaacuga |
| 106 | isomiR | mir-19a 3p | Mature 3' sub | 22 | ugugcaaaucuaugcaaaacug |
| 107 | isomiR | mir-19a 3p | Mature 3' sub | 21 | ugugcaaaucuaugcaaaacu |
| 108 | isomiR | mir-19b-1//mir-19b-2 3p | Mature 3' sub | 20 | ugugcaaauccaugcaaaac |
| 109 | isomiR | mir-19b-1//mir-19b-2 3p | Mature 3' sub | 21 | ugugcaaauccaugcaaaacu |
| 110 | miRNA | mir-20a 5p | Mature 5' | 23 | uaaagugcuuauagugcagguag |

**Table 1-5**

| SEQ ID NO: | Class | Archetype | Type | Length (nucleotides) | Sequence |
|---|---|---|---|---|---|
| 111 | isomiR | mir-20a 5p | Mature 5' sub | 22 | uaaagugcuuauagugcaggua |
| 112 | isomiR | mir-20a 5p | Mature 5' sub | 21 | uaaagugcuuauagugcaggu |
| 113 | miRNA | mir-20b 5p | Mature 5' | 23 | caaagugcucauagugcagguag |
| 114 | isomiR | mir-20b 5p | Mature 5' sub | 21 | caaagugcucauagugcaggu |
| 115 | isomiR | mir-223 3p | Mature 3' sub | 21 | gucaguuugucaaauacccca |
| 116 | isomiR | mir-223 3p | Mature 3' sub/super | 22 | gucaguuugucaaauaccccaa |
| 117 | isomiR | mir-223 3p | Mature 3' sub | 20 | ugucaguuugucaaauaccc |
| 118 | isomiR | mir-223 3p | Mature 3' sub | 21 | ugucaguuugucaaauacccc |
| 119 | isomiR | mir-223 3p | Mature 3' super | 23 | ugucaguuugucaaauaccccaa |
| 120 | miRNA | mir-223 3p | Mature 3' | 22 | ugucaguuugucaaauacccca |
| 121 | isomiR | mir-24-1//mir-24-2 3p | Mature 3' sub | 19 | uggcucaguucagcaggaa |
| 122 | miRNA | mir-24-1//mir-24-2 3p | Mature 3' | 22 | uggcucaguucagcaggaacag |
| 123 | isomiR | mir-25 3p | Mature 3' sub | 20 | cauugcacuugucucggucu |
| 124 | isomiR | mir-25 3p | Mature 3' sub | 21 | cauugcacuugucucggucug |
| 125 | miRNA | mir-26a-1//mir-26a-2 5p | Mature 5' | 22 | uucaaguaauccaggauaggcu |
| 126 | miRNA | mir-26b 5p | Mature 5' | 21 | uucaaguaauucaggauaggu |
| 127 | isomiR | mir-26b 5p | Mature 5' sub | 20 | uucaaguaauucaggauagg |
| 128 | miRNA | mir-29a 3p | Mature 3' | 22 | uagcaccaucugaaaucgguua |
| 129 | miRNA | mir-29c 3p | Mature 3' | 22 | uagcaccauuugaaaucgguua |
| 130 | isomiR | mir-29c 3p | Mature 3' sub | 21 | uagcaccauuugaaaucgguu |
| 131 | isomiR | mir-30d 5p | Mature 5' sub | 20 | uguaaacauccccgacugga |
| 132 | isomiR | mir-30e 5p | Mature 5' sub/super | 23 | guaaacauccuugacuggaagcu |
| 133 | isomiR | mir-30e 5p | Mature 5' super | 24 | uguaaacauccuugacuggaagcu |
| 134 | isomiR | mir-320a 3p | Mature 3' sub/super | 22 | aaagcuggguugagagggcgaa |
| 135 | isomiR | mir-342 3p | Mature 3' sub | 22 | ucucacacagaaaucgcacccg |
| 136 | miRNA | mir-342 3p | Mature 3' | 23 | ucucacacagaaaucgcacccgu |
| 137 | isomiR | mir-34a 5p | Mature 5' sub | 20 | gcagugucuuagcugguugu |

**Table 1-6**

| SEQ ID NO: | Class | Archetype | Type | Length (nucleotides) | Sequence |
|---|---|---|---|---|---|
| 138 | isomiR | mir-423 5p | Mature 5' sub | 19 | ugaggggcagagagcgaga |
| 139 | miRNA | mir-423 5p | Mature 5' | 23 | ugaggggcagagagcgagacuuu |
| 140 | miRNA | mir-425 5p | Mature 5' | 23 | aaugacacgaucacucccguuga |
| 141 | isomiR | mir-451a 5p | Mature 5' sub | 21 | aaccguuaccauuacugaguu |
| 142 | isomiR | mir-451 a 5p | Mature 5' sub | 20 | aaaccguuaccauuacugag |
| 143 | isomiR | mir-451a 5p | Mature 5' super | 25 | aaaccguuaccauuacugaguuuag |
| 144 | isomiR | mir-451a 5p | Mature 5' super | 24 | aaaccguuaccauuacugaguuua |
| 145 | isomiR | mir-451a 5p | Mature 5' sub | 17 | aaaccguuaccauuacu |
| 146 | isomiR | mir-451a 5p | Mature 5' super | 23 | aaaccguuaccauuacugaguuu |
| 147 | isomiR | mir-451a 5p | Mature 5' sub | 19 | aaaccguuaccauuacuga |
| 148 | isomiR | mir-451a 5p | Mature 5' sub | 21 | aaaccguuaccauuacugagu |
| 149 | miRNA | mir-451a 5p | Mature 5' | 22 | aaaccguuaccauuacugaguu |
| 150 | isomiR | mir-486-1//mir-486-2 5p | Mature 5' sub | 20 | uccuguacugagcugccccg |
| 151 | isomiR | mir-7-1//mir-7-2//mir-7-3 5p | Mature 5' sub | 21 | gaagacuagugauuuuguugu |
| 152 | isomiR | mir-7-1//mir-7-2//mir-7-3 5p | Mature 5' sub | 20 | gaagacuagugauuuuguug |
| 153 | miRNA | mir-92a-1//mir-92a-2 3p | Mature 3' | 22 | uauugcacuugucccggccugu |
| 154 | isomiR | mir-92a-1//mir-92a-2 3p | Mature 3' sub | 21 | uauugcacuugucccggccug |
| 155 | miRNA | mir-93 5p | Mature 5' | 23 | caaagugcuguucgugcagguag |
| 156 | isomiR | mir-93 5p | Mature 5' sub | 20 | caaagugcuguucgugcagg |
| 157 | isomiR | mir-93 5p | Mature 5' sub | 21 | caaagugcuguucgugcaggu |
| 158 | tRF | Homo_sapiens_tRNA-Ala-AGC-2-1//···* 12 | Exact | 30 | ggggguguagcucagugguagagcgcgugc |
| 159 | tRF | Homo_sapiens_tRNA-Glu-CTC-1-1//···*3 | Exact | 26 | ucccugguggucuagugguuaggauu |
| 160 | tRF | Homo_ sapiens_tRNA-Gly-CCC-2-1//···*13 | Exact | 31 | cgccgcugguguagugguaucaugcaagauu |
| 161 | tRF | Homo_ sapiens_tRNA-Gly-CCC-2-1//···*13 | Exact | 29 | cgccgcugguguagugguaucaugcaaga |
| 162 | tRF | Homo_ sapiens_tRNA-Gly-CCC-2-1//···*13 | Exact | 30 | cgccgcugguguagugguaucaugcaagau |
| 163 | tRF | Homo_ sapiens_tRNA-Gly-CCC-2-1//···*13 | Exact | 30 | gcgccgcugguguagugguaucaugcaaga |
| 164 | tRF | Homo_ sapiens_tRNA-Gly-CCC-2-1//···*13 | Exact | 26 | gcgccgcugguguagugguaucaugc |
| 165 | tRF | Homo_ sapiens_tRNA-Gly-CCC-2-1//···*13 | Exact | 22 | gcgccgcugguguagugguauc |
| 166 | tRF | Homo_sapiens_tRNA-Gly-CCC-2-1//···*13 | Exact | 27 | gcgccgcugguguagugguaucaugca |
| 167 | tRF | Homo_ sapiens_tRNA-Gly-GCC-1-1//···*4 | Exact | 25 | gcaugggugguucagugguagaauu |

**Table 1-7**

| SEQ ID NO: | Class | Archetype | Type | Length (nucleotides) | Sequence |
|---|---|---|---|---|---|
| 168 | tRF | Homo_ sapiens_tRNA-iMet-CAT-1-1//···*2 | Exact | 30 | agcagaguggcgcagcggaagcgugcuggg |
| 169 | tRF | Homo_sapiens_tRNA-iMet-CAT-1-1//···*2 | Exact | 29 | agcagaguggcgcagcggaagcgugcugg |
| 170 | tRF | Homo_ sapiens_tRNA-Lys-CTT-1-1//···*7 | Exact | 31 | gcccggcuagcucagucgguagagcauggga |
| 171 | tRF | Homo_sapiens_tRNA-Lys-CTT-1-1//···*7 | Exact | 32 | gcccggcuagcucagucgguagagcaugggac |
| 172 | tRF | Homo_sapiens_tRNA-Val-AAC-1-1//···*5 | Exact | 31 | guuuccguaguguagugguuaucacguucgc |
| 173 | tRF | tRNA-Val-CAC-3-1 ···*14 | Exact | 31 | guuuccguaguguagcgguuaucacauucgc |
| 174 | tRF | Homo_sapiens_tRNA-Gly-CCC-1-1//···*8 | Exact | 30 | gcauuuuucagugguagaauucucgc |
| 175 | LincRNA | ENST00000229465.10//···*24 | Exact | 17 | cacaugaaaaaaugcuc |
| 176 | LincRNA | ENST00000229465.10//···*15 | Exact | 15 | caugaaaaaaugcuc |
| 177 | RRNA | ENST00000616292.1//···*17 | Exact | 17 | gacucuuagcgguggau |
| 178 | tRF | Homo_ sapiens_tRNA-Gly-CCC-2-1//···*13 | Exact | 29 | ccgcugguguagugguaucaugcaagauu |
| 179 | snoRNA | ENST00000580533.1//···*25 | Exact | 23 | ggagagaacgcggucugaguggu |
| 180 | RRNA | ENST00000616292.1//···*19 | Exact | 16 | gacucuuagcggugga |
| 181 | snoRNA | ENST00000580533.1//···*16 | Exact | 28 | gagagggagagaacgcggucugaguggu |
| 182 | snoRNA | ENST00000580533.1//···*26 | Exact | 27 | agagggagagaacgcggucugaguggu |
| 183 | isomiR | mir-145 | Mature 5' sub | 18 | guccaguuuucccaggaa |
| 184 | isomiR | mir-223 | Mature 3' sub | 19 | ugucaguuugucaaauacc |
| 185 | precursor | mir-145 | Precursor | 17 | guccaguuuucccagga |
| 186 | isomiR | mir-23a//mir-23b | Mature 3' sub | 17 | aucacauugccagggau |
| 187 | tRF | Homo_sapiens_tRNA-Gly-CCC-2-1//···*13 | Exact | 24 | gguguagugguaucaugcaagauu |
| 188 | isomiR | mir-122 | Mature 5' sub | 19 | uggagugugacaauggugu |
| 189 | isomiR | mir-27a//mir-27b | Mature 3' sub | 18 | uucacaguggcuaaguuc |
| 190 | isomiR | mir-145 | Mature 5' sub | 20 | guccaguuuucccaggaauc |
| 191 | RRNA | ENST00000616292.1//···*21 | Exact | 15 | gacucuuagcggugg |
| 192 | isomiR | mir-99a | Mature 5' sub | 21 | aacccguagauccgaucuugu |
| 193 | isomiR | mir-142 | Mature 3' sub | 22 | uguaguguuuccuacuuuaugg |
| 194 | tRF | Homo_ sapiens_tRNA-Gly-CCC-2-1//···*13 | Exact | 27 | gcugguguagugguaucaugcaagauu |
| 195 | isomiR | mir-145 | Mature 5' sub | 19 | guccaguuuucccaggaau |
| 196 | isomiR | mir-122 | Mature 5' sub | 20 | uggagugugacaaugguguu |
| 197 | isomiR | mir-30a | Mature 5' sub | 21 | uguaaacauccucgacuggaa |

**Table 1-8**

| SEQ ID NO: | Class | Archetype | Type | Length (nucleotides) | Sequence |
|---|---|---|---|---|---|
| 198 | isomiR | mir-27b | Mature 3' sub | 20 | uucacaguggcuaaguucug |
| 199 | isomiR | mir-23b | Mature 3' super | 22 | aucacauugccagggauuacca |
| 200 | tRF | Homo_ sapiens_tRNA-Ser-AGA-1-1//···*33 | Exact | 25 | guagucguggccgagugguuaaggc |
| 201 | MiscRNA | ENST00000363745 | Exact | 23 | cccccacugcuaaauuugacugg |
| 202 | tRF | Homo_ sapiens_tRNA-Val-AAC-1-1//···*5 | Exact | 18 | guuuccguaguguagugg |
| 203 | tRF | Homo_sapiens_tRNA-Glu-TTC-2-1//···*31 | Exact | 26 | ucccacauggucuagcgguuaggauu |
| 204 | tRF | Homo_ sapiens_tRNA-Pro-AGG-1-1//···*34 | Exact | 25 | ggcucguuggucuagggguaugauu |
| 205 | isomiR | mir-451a | Mature 5' sub | 19 | aaccguuaccauuacugag |
| 206 | MiscRNA | ENST00000363745.1//···*22 | Exact | 24 | ccccccacugcuaaauuugacugg |
| 207 | isomiR | mir-106a | Mature 5' sub | 22 | aaaagugcuuacagugcaggua |
| 208 | miRNA | mir-652 | Mature 3' | 21 | aauggcgccacuaggguugug |
| 209 | tRF | Homo_ sapiens_tRNA-Val-CAC-3-1 | Exact | 32 | guuuccguaguguagcgguuaucacauucgcc |
| 210 | tRF | Homo_sapiens_tRNA-Glu-CTC-1-1//···*3 | Exact | 23 | ucccugguggucuagugguuagg |
| 211 | tRF | Homo_ sapiens_tRNA-Lys-CTT-1-1//···*10 | Exact | 28 | gcccggcuagcucagucgguagagcaug |
| 212 | isomiR | mir-103a-2//mir-103a-1 | Mature 3' sub | 22 | agcagcauuguacagggcuaug |
| 213 | tRF | Homo_ sapiens_tRNA-Gly-GCC-1-1//···*4 | Exact | 24 | gcaugggugguucagugguagaau |
| 214 | tRF | Homo_ sapiens_tRNA-Lys-CTT-2-1//···*6 | Exact | 30 | gcccggcuagcucagucgguagagcaugag |
| 215 | miRNA | mir-454 | Mature 3' | 23 | uagugcaauauugcuuauagggu |
| 216 | isomiR | mir-486-1//mir-486-2 | Mature 5' sub | 18 | uccuguacugagcugccc |
| 217 | tRF | Homo_ sapiens_tRNA-Gly-GCC-1-1//···*11 | Exact | 23 | gcaugggugguucagugguagaa |
| 218 | isomiR | mir-550a-1//mir-550a-2//mir-550a-3 | Mature 3' sub | 21 | ugucuuacucccucaggcaca |
| 219 | precursor | mir-486-1//mir-486-2 | Precursor | 16 | uccuguacugagcugc |
| 220 | isomiR | mir-93 | Mature 5' sub | 22 | caaagugcuguucgugcaggua |
| 221 | tRF | Homo_ sapiens_tRNA-Val-CAC-3-1 | Exact | 23 | guuuccguaguguagcgguuauc |
| 222 | tRF | Homo_sapiens_tRNA-Pro-AGG-1-1//···34 | Exact | 27 | ggcucguuggucuagggguaugauucu |
| 223 | tRF | Homo_sapiens_tRNA-Glu-TTC-2-1//···*31 | Exact | 28 | ucccacauggucuagcgguuaggauucc |
| 224 | tRF | Homo_ sapiens_tRNA-Ser-GCT-1-1//···*32 | Exact | 25 | gacgagguggccgagugguuaaggc |
| 225 | isomiR | mir-45 1a | Mature 5' sub/super | 24 | aaccguuaccauuacugaguuuag |
| 226 | tRF | Homo_ sapiens_tRNA-Ser-GCT-1-1//···*32 | Exact | 24 | gacgagguggccgagugguuaagg |

**Table 1-9**

| SEQ ID NO: | Class | Archetype | Type | Length (nucleotides) | Sequence |
|---|---|---|---|---|---|
| 227 | isomiR | mir-7-1//mir-7-2//mir-7-3 | Mature 5' sub | 22 | uggaagacuagugauuuuguug |
| 228 | tRF | Homo_sapiens_tRNA-Cys-GCA-2-1//···*30 | Exact | 32 | ggggguauagcucagugguagagcauuugacu |
| 229 | tRF | Homo sapiens_tRNA-Ser-AGA-1-1//···*33 | Exact | 23 | guagucguggccgagugguuaag |
| 230 | tRF | Homo_sapiens_tRNA-SeC-TCA-1-1 | Exact | 34 | gcccggaugauccucaguggucuggggugcaggc |
| 231 | tRF | Homo_ sapiens_tRNA-Ser-AGA-1-1//···*33 | Exact | 24 | guagucguggccgagugguuaagg |
| 232 | isomiR | mir-20b | Mature 5' sub | 22 | caaagugcucauagugcaggua |
| 233 | MiscRNA | ENST00000364228.1//···* 18 | Exact | 23 | ggcugguccgaagguagugaguu |
| 234 | isomiR | mir-106b | Mature 3' sub/super | 22 | uaccgcacuguggguacuugcu |
| 235 | tRF | Homo_ sapiens_tRNA-Glu-CTC-1-1//···*3 | Exact | 27 | ucccugguggucuagugguuaggauuc |
| 236 | tRF | Homo sapiens tRNA-Val-TAC-1-1//···*28 | Exact | 23 | gguuccauaguguagugguuauc |
| 237 | tRF | Homo_ sapiens_tRNA-Glu-CTC-1-1//···*3 | Exact | 24 | ucccugguggucuagugguuagga |
| 238 | tRF | Homo_ sapiens_tRNA-Ala-AGC-8-1//···*27 | Exact | 23 | gggggauuagcucaaaugguaga |
| 239 | MiscRNA | ENST00000363667.1//···*20 | Exact | 18 | gcuaaauuugacuggcuu |
| 240 | tRF | Homo_ sapiens_ tRNA-Glu-TTC-2-1//···*31 | Exact | 29 | ucccacauggucuagcgguuaggauuccu |
| 241 | tRF | Homo sapiens_tRNA-Val-CAC-2-1 | Exact | 23 | gcuucuguaguguagugguuauc |
| 242 | tRF | Homo_ sapiens_tRNA-SeC-TCA-1-1 | Exact | 29 | gcccggaugauccucaguggucuggggug |
| 243 | tRF | Homo_sapiens_tRNA-Cys-GCA-2-1//···*30 | Exact | 31 | ggggguauagcucagugguagagcauuugac |
| 244 | isomiR | mir-324 | Mature 5' sub | 21 | cgcauccccuagggcauuggu |
| 245 | tRF | Homo sapiens_tRNA-Phe-GAA-1-1//···*35 | Exact | 25 | gccgaaauagcucaguugggagagc |
| 246 | tRF | Homo_sapiens_tRNA-Ser-GCT-1-1//···*32 | Exact | 23 | gacgagguggccgagugguuaag |
| 247 | tRF | Homo sapiens_tRNA-Val-AAC-1-//···*5 | Exact | 28 | guuuccguaguguagugguuaucacguu |
| 248 | tRF | Homo_ sapiens_tRNA-Lys-CTT-1-1//···*10 | Exact | 26 | gcccggcuagcucagucgguagagca |
| 249 | tRF | Homo_ sapiens_tRNA-Lys-CTT-1-1//···*7 | Exact | 25 | gcccggcuagcucagucgguagagc |
| 250 | tRF | Homo_ sapiens_tRNA-Val-AAC-1-1//···*5 | Exact | 30 | guuuccguaguguagugguuaucacguucg |
| 251 | tRF | Homo_sapiens_tRNA-Val-AAC-1-1//···*5 | Exact | 23 | guuuccguaguguagugguuauc |
| 252 | tRF | Homo_ sapiens_tRNA-Glu-CTC-1-1//···*3 | Exact | 25 | ucccugguggucuagugguuaggau |
| 253 | tRF | Homo_ sapiens_tRNA-Glu-CTC-1-1//···*3 | Exact | 29 | ucccugguggucuagugguuaggauucgg |
| 254 | tRF | Homo_sapiens_tRNA-Ala-AGC-8-1//···*27 | Exact | 25 | gggggauuagcucaaaugguagagc |
| 255 | tRF | Homo_ sapiens_tRNA-Val-TAC-1-1//···*28 | Exact | 26 | gguuccauaguguagugguuaucacg |

**Table 1-10**

| SEQ ID NO: | Class | Archetype | Type | Length (nucleotides) | Sequence |
|---|---|---|---|---|---|
| 256 | tRF | Homo_sapiens_ tRNA-Cys-GCA-2-1//···*30 | Exact | 31 | gggguauagcucagugguagagcauuugacu |
| 257 | tRF | Homo_sapiens_tRNA-Ala-AGC-8-1//···*27 | Exact | 24 | gggggauuagcucaaaugguagag |
| 258 | tRF | Homo_sapiens_ tRNA-Trp-CCA-3-1//···*29 | Exact | 24 | gaccucguggcgcaacgguagcgc |
| 259 | tRF | Homo_ sapiens_tRNA-Val-AAC-1-1//···*5 | Exact | 26 | guuuccguaguguagugguuaucacg |
| 260 | tRF | Homo_ sapiens_tRNA-Val-AAC-1-1//···*5 | Exact | 24 | guuuccguaguguagugguuauca |
| 261 | tRF | Homo_sapiens_tRNA-Ala-AGC-11-1 | Exact | 25 | ggggaauuagcucaaaugguagagc |
| 262 | tRF | Homo_sapiens_tRNA-Val-AAC-1-1//···*5 | Exact | 29 | guuuccguaguguagugguuaucacguuc |
| 263 | tRF | Homo_ sapiens_tRNA-Trp-CCA-2-1 | Exact | 24 | gaccucguggcgcaaugguagcgc |
| 264 | tRF | Homo_sapiens_tRNA-Ala-AGC-8-1//···*27 | Exact | 24 | ggggauuagcucaaaugguagagc |
| 265 | tRF | Homo_ sapiens tRNA-Val-AAC-1-1//···*5 | Exact | 25 | guuuccguaguguagugguuaucac |
| 266 | isomiR | mir-21 5p | Mature 5' super | 23 | uagcuuaucagacugauguugac |
| 267 | isomiR | mir-23a 3p | Mature 3' super | 22 | aucacauugccagggauuucca |
| 268 | isomiR | mir-27a 3p | Mature 3' sub | 20 | uucacaguggcuaaguuccg |
| 269 | MiscRNA | ENST00000364600.1//···*36 | Exact | 28 | ggcugguccgaugguaguggguuaucag |

*1: Homo_sapiens_tRNA-Gly-CCC-1-1//Homo_sapiens_tRNA-Gly-CCC-1-2//Homo_sapiens_tRNA-Gly-GCC-2-1//Homo_sapiens_tRNA-Gly-GCC-2-2//Homo_sapiens_tRNA-Gly-GCC-2-3//Homo_sapiens_tRNA-Gly-GCC-2-4//Homo_sapiens_tRNA-Gly-GCC-2-5//Hom o_sapiens_tRNA-Gly-GCC-2-6//omo_sapiens_tRNA-Gly-GCC-3-1//Homo_sapiens_tRNA-Gly-GCC-5-1
*2: Homo_sapiens_tRNA-iMet-CAT-1-1//Homo_sapiens_tRNA-iMet-CAT-1-2//Homo_sapiens_tRNA-iMet-CAT-1-3//Homo_sapiens_tRNA-iMet-CAT-1-4//Homo_sapiens_tRNA-iMet-CAT-1-5//Homo_sapiens_tRNA-iMet-CAT-1-6//Homo_sapiens_tRNA-iMet-CAT-1-7//Homo_sapiens_tRNA-iMet-CAT-1-8//Homo_sapiens_tRNA-iMet-CAT-2-1
*3: Homo_sapiens_tRNA-Glu-CTC-1-1//Homo_sapiens_tRNA-Glu-CTC-1-2//Homo_sapiens_tRNA-Glu-CTC-1-3//Homo_sapiens_tRNA-Glu-CTC-1-4//Homo_sapiens_tRNA-Glu-CTC-1-5//Homo_sapiens_tRNA-Glu-CTC-1-6//Homo_sapiens_tRNA-Glu-CTC-1-7//Homo_sapiens_tRNA-Glu-CTC-2-1
*4: Homo_sapiens_tRNA-Gly-GCC-1-1//Homo_sapiens_tRNA-Gly-GCC-1-2//Homo_sapiens_tRNA-Gly-GCC-1-3//Homo-sapiens_tRNA-Gly-GCC-1-4//Homo_sapiens_tRNA-Gly-GCC-1-5
*5: Homo_sapiens_tRNA-Val-AAC-1-1//Homo_sapiens_tRNA-Val-AAC-1-2//Homo_sapiens_tRNA-Val-AAC-1-3//Homo_sapiens_tRNA-Val-AAC-1-4//Homo_sapiens_tRNA-Val-AAC-1-5//Homo_sapiens_tRNA-Val-AAC-3-1//Homo_sapiens_tRNA-Val-AAC-4-1//Homo_sapiens_tRNA-Val-CAC-1-1//Homo_sapiens_tRNA-Val-CAC-1-2//Homo_sapiens_tRNA-Val-CAC-1-3//Homo_sapiens_tRNA-Val-CAC-1-4//Homo_sapiens_tRNA-Val-CAC-1-5//Homo_sapiens_tRNA-Val-CAC-1-6//Homo_sapiens_tRNA-Val-CAC-4-1//Homo_sapiens_tRNA-Val-CAC-5-1
*6: Homo_sapiens_tRNA-Lys-CTT-2-1//Homo_sapiens_tRNA-Lys-CTT-2-2//Homo_sapiens_tRNA-Lys-CTT-2-3//Homo_sapiens_tRNA-Lys-CTT-2-4//Homo_sapiens_tRNA-Lys-CTT-2-5//Homo_sapiens_tRNA-Lys-CTT-3-1
*7: Homo_sapiens_tRNA-Lys-CTT-1-1//Homo_sapiens_tRNA-Lys-CTT-1-2//Homo_sapiens_tRNA-Lys-CTT-4-1
*8: Homo_sapiens_tRNA-Gly-CCC-1-1//Homo_sapiens_tRNA-Gly-CCC-1-2//Homo_sapiens_tRNA-Gly-GCC-2-1//Homo_sapiens_tRNA-Gly-GCC-2-2//Homo_sapiens_tRNA-Gly-GCC-2-3//Homo_sapiens_tRNA-Gly-GCC-2-4//Homo_sapiens_tRNA-Gly-GCC-2-5//Homo_sapiens_tRNA-Gly-GCC-2-6//Homo_sapiens-tRNA-Gly-GCC-3-1//Homo_sapiens_tRNA-Gly-GCC-5-1
*9: Homo_sapiens_tRNA-Ala-AGC-4-1//Homo_sapiens_tRNA-Ala-CGC-1-1//Homo_sapiens_tRNA-Ala-CGC-2-1//Homo_sapiens_tRNA-Ala-TGC-2-1//Homo_sapiens_tRNA-Ala-TGC-3-1//Homo-sapiens_tRNA-Ala-TGC-3-2//Homo_sapiens_tRNA-Ala-TGC-4-1
*10: Homo_sapiens_tRNA-Lys-CTT-1-1//Homo_sapiens_tRNA-Lys-CTT-1-2//Homo_sapiens_tRNA-Lys-CTT-4-1
*11: Homo_sapiens_tRNA-Gly-GCC-1-1//Homo_sapiens_tRNA-Gly-GCC-1-2//Homo_sapiens_tRNA-Gly-GCC-1-3//Homo_sapiens_tRNA-Gly-GCC-1-4//Homo_sapiens_tRNA-Gly-GCC-1-5
*12: Homo_sapiens_tRNA-Ala-AGC-2-1//Homo_sapiens_tRNA-Ala-AGC-2-2//Homo_sapiens_tRNA-Ala-AGC-3-1//Homo_sapiens_tRNA-Ala-AGC-5-1//Homo_sapiens_tRNA-Ala-AGC-7-1//Homo_sapiens_tRNA-Ala-CGC-4-1
*13: Homo_sapiens_tRNA-Gly-CCC-2-1//Homo_sapiens_tRNA-Gly-CCC-2-2
*14: Homo_sapiens_tRNA-Val-CAC-3-1
*15: ENST00000229465.10//ENST00000392385.2//ENST00000505089.6//ENST00000454224.1//ENST00000625513.1//ENST00000612496.1//ENST00000612997.1//ENST00000623130.1//ENST00000597346.1//ENST00000554008.5//ENST00000511895.1//ENST00000507761.1//ENST00000589496.2
*16: ENST00000580533.1//ENST00000625845.1//ENST00000620446.1//ENST00000577988.2//ENST00000631292.1//ENST00000617128.1//ENST00000571722.3//ENST00000364880.2//ENST00000628329.1//ENST00000619178.1//ENST00000584923.1//ENST00000625876.1//ENST00000620232.1//ENST00000630092.1//ENST00000573866.2
*17: ENST00000616292.1//ENST00000610460.1//ENST00000618998.1//ENST00000619779.1//ENST00000611446.1//ENST00000612463.1//ENST00000619471.1//ENST00000613359.1
*18: ENST00000364228.1//ENST00000365403.1
*19: ENST00000616292.1//ENST00000610460.1//ENST00000618998.1//ENST00000619779.1//ENST00000611446.1//ENST00000612463.1//ENST00000619471.1//ENST00000613359.1
*20: ENST00000363667.1//ENST00000363745.1//ENST00000365281.1//ENST00000364600.1//ENST00000365436.1//ENST00000391023.1//ENST00000364678.1//ENST00000516225.1//ENST00000611372.//ENST00000364338.1//ENST00000364409.1//ENST00000516507.//ENST00000391107.1//ENST00000459254.1//ENST00000364507.1//ENST00000363341.1
*21: ENST00000616292.1//ENST00000610460.1//ENST00000618998.1//ENST00000619779.1//ENST00000611446.1//ENST00000612463.1//ENST00000619471.1//ENST00000613359.1
*22: ENST00000363745.1//ENST00000459091.1//ENST00000364409.1//ENST00000516507.1
*23: ENST00000363745.1//ENST00000459091.1//ENST00000364409.1//ENST00000516507.1//ENST00000391107.1//ENST00000459254.1
*24: ENST00000229465,10//ENST00000392385.2//ENST00000505089.6//ENST00000454224.1//ENST00000625513.1//ENST00000612496.1//ENST00000612997.1//ENST00000623130.1//ENST00000597346.1//ENST00000511895.1//ENST00000507761.1//ENST00000589496.2
*25: ENST00000580533.1//ENST00000625845.1//ENST00000620446.1//ENST00000577988.2//ENST0000063292.1//ENST00000617128.1//ENST00000571722.3//ENST00000364880.2//ENST00000628329.1//ENST00000619178.1//ENST00000584923.1//ENST00000625876.1//ENST00000620232.1//ENST00000630092.1//ENST00000573866.2
*26: ENST00000580533.1//ENST00000625845.1//ENST00000620446.1//ENST00000577988.2//ENST00000631292.1//ENST0000067128.//ENST00000571722.3//ENST00000364880.2//ENST00000628329.1//ENST00000619178.1//ENST00000584923.1//ENST00000625876.1//ENST00000620232.1//ENST00000630092.1//ENST0000057866.2
*27: Homo_sapiens_tRNA-Ala-AGC-8-1//Homo_sapiens_tRNA-Ala-AGC-8-2
*28: Homo_sapiens_tRNA-Val-TAC-1-1//Homo_sapiens_tRNA-Val-TAC-1-2
*29: Homo_sapiens_tRNA-Trp-CCA-3-1//Homo_sapiens_tRNA-Trp-CCA-3-2//Homo_sapiens_tRNA-Trp-CCA-3-3//Homo-sapiens_tRNA-Trp-CCA-4-1
*30: Homo-sapiens_tRNA-Cys-GCA-2-1//Homo_sapiens-tRNA-Cys-GCA-2-2//Homo_sapiens_tRNA-Cys-GCA-2-3//Homo_sapiens_tRNA-Cys-GCA-2-4//Homo_sapiens_tRNA-Cys-GCA-4-1//Homo_sapiens_tRNA-Cys-GCA-chr5-2
*31: Homo_sapiens_tRNA-Glu-TTC-2-1//Homo_sapiens_tRNA-Glu-TTC-2-2
*32: Homo_sapiens_tRNA-Ser-GCT-1-1//Homo_sapiens_tRNA-Ser-GCT-2-1//Homo_sapiens_tRNA-Ser-GCT-3-1//Homo_sapiens_tRNA-Ser-GCT-4-1//Homo_sapiens_tRNA-Ser-GCT-4-2//Homo_sapiens_tRNA-Ser-GCT-4-3//Homo_sapiens_tRNA-Ser-GCT-5-
*33: Homo_sapiens_tRNA-Ser-AGA-1-1//Homo_sapiens_tRNA-Ser-AGA-2-1//Homo_sapiens_tRNA-Ser-AGA-2-2//Homo_sapiens_tRNA-Ser-AGA-2-3//Homo_sapiens_tRNA-Ser-AGA-2-4//Homo_sapiens_tRNA-Ser-AGA-2-5//Homo_sapiens_tRNA-Ser-AGA-2-6//Homo_sapiens_tRNA-Ser-AGA-3-1//Homo_sapiens_tRNA-Ser-AGA-4-1//Homo_sapiens_tRNA-Ser-TGA-2-1//Homo_sapiens_tRNA-Ser-TGA-3-1//Homo_sapiens_tRNA-Ser-TGA-4-1
*34: Homo_sapiens_tRNA-Pro-AGG-1-1//Homo_sapiens_tRNA-Pro-AGG-2-1//Homo_sapiens_tRNA-Pro-AGG-2-2//Homo_sapiens_tRNA-Pro-AGG-2-3//Homo_sapiens_tRNA-Pro-AGG-2-4//Homo_sapiens_tRNA-Pro-AGG-2-5//Homo_sapiens_tRNA-Pro-AGG-2-6//Homo_sapiens_tRNA-Pro-AGG-2-7//Homo_sapiens_tRNA-Pro-ACG-2-8//Homo_sapiens_tRNA-Pro-CGG-1-1//Homo_sapiens_tRNA-Pro-CGG-1-2//Homo_sapienstRNA-Pro-CGG-1-3//Homo_sapiens_tRNA-Pro-CGG-2-1//Homo_sapiens_tRNA-Pro-TGG-2-1//Homo_sapiens_tRNA-Pro-TGG-3-1//Homo_sapiens_tRNA-Pro-TGG-3-2//Homo_sapiens_tRNA-Pro-TGG-3-3//Homo_sapiens_tRNA-Pro-TGG-3-4//Homo_sapiens_tRNA-Pro-TGG-3-5
*35: Homo_sapiens_tRNA-Phe-GAA-1-1//tRNA-Phe-GAA--2//tRNA-Phe-GAA-1-3//tRNA-Phe-GAA--4//tRNA-Phe-GAA-1-5//tRNA-Phe-GAA-1-6//tRNA-Phe-GAA-2-1//tRNA-Phe-GAA-4-1
*36: ENST00000364600.1//ENST00000577883.2//ENST00000577984.2//ENST00000516678.1//ENST00000516507.1//ENST00000481041.3//ENST000005725.2//ENST00000365571.2//ENST00000578877.2//ENST00000364908.1

Among those miRNAs or the like, miRNAs or the like whose nucleotide sequences are represented by SEQ ID NOs: 1 to 33, 56 to 173, and 175 to 269 (for example, "a miRNA or the like whose nucleotide sequence is represented by SEQ ID NO: 1" is hereinafter sometimes referred to simply as "a miRNA or the like represented by SEQ ID NO: 1" or "one represented by SEQ ID NO: 1" for convenience) are present in serum, and those represented by SEQ ID NOs: 34 to 55, and 174 are present in exosomes in serum.

Many of those miRNAs or the like show the logarithm of the ratio of the abundance in serum or exosomes from patients with breast cancer to the abundance in serum or exosomes from healthy subjects (represented by "log FC," which means the logarithm of FC (fold change) to base 2) is more than 0.585 in absolute value (that is, a ratio of not less than about 1.5 or not more than about 1/1.5), which is statistically significant (t-test; *p* < 0.05).

The abundance of miRNAs or the like represented by SEQ ID NOs: 1 to 19, 27, 28, 34 to 51, 74, 76, 77, 80 to 84, 96, 101 to 104, 115 to 122, 125, 128, 134 to 139, 151, 152, 159 to 165, 168, 169, and 175 to 199 is higher in patients with breast cancer than in healthy subjects, while the abundance of miRNAs or the like represented by SEQ ID NOs: 20 to 26, 29 to 33, 52 to 54, 56 to 73, 75, 78 to 79, 85 to 95, 97 to 100, 105 to 114, 123, 124, 126, 127, 129 to 133, 140 to 150, 153 to 158, 166, 167, 170 to 173, and 200 to 269 is lower in patients with breast cancer than in healthy subjects.

Among those, the miRNAs or the like whose nucleotide sequences are represented by any of SEQ ID NOs: 2. 21, 22, 23, 24, 26, 30, 31, 32, 33, 34, 152, 151, 15, 28, 41, 1, 14, 27, 40, 25, 12, 160, 3 to 11, 13, 16, 20, 27, 29, 37 to 39, 41, 43, 45, 47 to 52, 56, 60, 66, 82, 86, 90 to 92, 107, 111, 112, 126, 127, 130, 137, 158, 161, 162, 173, and 175 to 265 have a log FC value of not less than 1.5 in absolute value and thus function as indexes with especially high sensitivity, and are preferable.

Additionally, among these, even stage 0 breast cancer (that is, cancer which is at a stage when no tumor mass has been formed and is undetectable by diagnostic imaging or palpation) can be detected by a method in which the abundance of one represented by any one of SEQ ID NOs: 3 to 9 is used as an index, as specifically described in Examples below.

The accuracy of each cancer marker is indicated using the area under the ROC curve (AUC: Area Under Curve) as an index, and cancer markers with an AUC value of 0.7 or higher are generally considered effective. AUC values of 0.90 or higher, 0.97 or higher, 0.98 or higher, and 1.00 correspond to cancer markers with high accuracy, very high accuracy, even higher accuracy, and complete accuracy (with no false-positive and false-negative events), respectively. Thus, the AUC value of each cancer marker is likewise preferably 0.90, more preferably not less than 0.97, still more preferably not less than 0.98, yet more preferably not less than 0.99. and most preferably 1.00 in the present invention. Those whose nucleotide sequences are represented by SEQ ID NOs: 2, 21, 22, 23, 24, 26, 30, 31, 32, 33, 34, 152, 151, 15, 28, 41, 1, 14, 27, 40, 25, 12, and 160 are preferable because of an AUC value of 0.97 or higher; among those, those represented by SEQ ID NOs: 2, 21, 22, 23, 24, 26, 30. 31, 32, 33, 34, 152, 151, 15, 28, 41, and 1 are more preferable because of an AUC value of 0.98 or higher; those represented by SEQ ID NOs: 2, 21, 22, 23, 24, 26, 30, 31, 32, 33, 34, and 152 are most preferable because of an AUC value of 1.00.

Furthermore, the abundance of the miRNAs or the like whose nucleotide sequences are represented by SEQ ID NOs: 2, 21, 22, 23, 24, 26, 31 to 33, and 55 is zero in either cancer patients or healthy subjects, and use of those miRNAs or the like thus enables high accuracy detection, similarly to miRNAs or the like having an AUC value of 1.00 (most of the small RNAs also have an AUC value of 1.00).

The test sample is not specifically limited, provided that the test sample is a body fluid containing miRNAs; typically, it is preferable to use a blood sample (including plasma, serum, and whole blood). For those represented by SEQ ID NOs: 1 to 33, 56 to 173, and 175 to 265, which are present in serum, it is simple and preferable to use serum or plasma as a test sample. For those represented by SEQ ID NOs: 34 to 54, which are present in exosomes, it is preferable to use serum or plasma as a test sample, to extract total RNA from the exosomes contained therein, and to measure the abundance of each miRNA or the like. The method of extracting total RNA in serum or plasma is well known and is specifically described in Examples below. The method *per se* of extracting total RNA from exosomes in serum or plasma is known and is specifically described in more detail in Examples below.

The abundance of each miRNA or the like is preferably measured (quantified) using a next-generation sequencer. Any instrument may be used and is not limited to a specific type of instrument, provided that the instrument determines sequences, similarly to next-generation sequencers. In the method of the present invention, as specifically described in Examples below, use of a next-generation sequencer is preferred over quantitative reverse-transcription PCR (qRT-PCR) which is widely used for quantification of miRNAs, to perform measurements from the viewpoint of accuracy because miRNAs or the like to be quantified include, for example, isomiRs, in which only one or more nucleotides are deleted from or added to the 5' and/or 3' ends of the original mature miRNAs thereof, and which should be distinguished from the original miRNAs when measured. Briefly, though details will be described specifically in Examples below, the quantification method can be performed, for example, as follows. When the RNA content in serum or plasma is constant, among reads measured in a next-generation sequencing analysis of the RNA content, the number of reads for each isomiR or mature miRNA per million reads is considered as the measurement value, where the total counts of reads with human-derived sequences are normalized to one million reads. When the RNA content in serum or plasma is variable in comparison with healthy subjects due to a disease, miRNAs showing little abundance variation in serum and plasma may be used. In cases where the abundance of miRNAs or the like in serum or plasma is measured, at least one miRNA selected from the group consisting of let-7g-5p, miR-425-3p, miR-425-5p, miR-23a-3p, miR-484-5p, and miR-191-5p is preferably used as an internal control, which are miRNAs showing little abundance variation in serum and plasma.

The cut-off value for the abundance of each miRNA or the like for use in evaluation is preferably determined based on the presence or absence of a statistically significant difference (*t*-test; *p* < 0.05, preferab*l*y *p* < 0.01, more preferably *p* < 0.001) from healthy subjects with regard to the abundance of the miRNA or the like. Specifically, the value of log₂ read counts (the cut-off value) can be preferably determined for each miRNA or the like, for example, at which the false-positive rate is optimal (the lowest); for example, the cut-off values (the values of log₂ read counts) for several miRNAs or the like are as indicated in Table 2. The cut-off values indicated in Table 2 are only examples, and other values may be employed as cut-off values as long as those values are appropriate to determine statistically significant difference. Additionally, the optimal cut-off values vary among different populations of patients and healthy subjects from which data is collected. However, a cut-off value may be set such that the cut-off value is within the range of, usually ±20%, particularly ±10%, from the cut-off value indicated in Table 2 or 3.

Additionally, as seen in Examples and Comparative Examples below, the abundance of a miRNA and that of each isomiR thereof are different between patients and healthy subjects, even among miRNAs or the like derived from the same archetype. For example, when miR-15a 5p is an archetype miRNA in Example 2 and Comparative Example 1 below, the log FC value of a miRNA (SEQ ID NO: 270) in Comparative Example 1 is 0, while the log FC value of an isomiR in the Mature-5'-sub type (SEQ ID NO: 2) in Example 2 is 5.67, indicating a predominantly higher abundance of the isomiR in patients with breast cancer. Thus, the measurement of the molecules represented by SEQ ID NO: 2 and SEQ ID NO: 270 in one patient can assist in breast cancer detection based on the abundance ratio thereof. Furthermore, Examples 85 to 88 (SEQ ID NOs: 85 to 88) are likewise isomiRs belonging to the miR-15a 5p family and each have different log FC values. Thus, the ratios between these values can be included into indexes to assist in more accurate detection. Because small differences in nucleotide sequence should be accurately distinguished, when the abundance of a certain miRNA and that of an isomiR thereof are measured, use of a next-generation sequencer is preferred over quantitative reverse-transcription PCR (qRT-PCR) which is typically used in miRNA measurement to perform measurements. Although no difference can be detected in the miRNA (SEQ ID NO: 270) in Comparative Example 1 which is a mature microRNA that can be detected by qRT-PCR, a significant difference can be found in Example 2 (SEQ ID NO:2) with the isomiR in the Mature-5'-sub type which can be detected by next-generation sequencers. Thus, using a next-generation sequencer is advantageous.

Each of the above miRNAs or the like is statistically significantly different in abundance between patients with breast cancer and healthy subjects, and may thus be used alone as an index. However, a combination of multiple miRNAs may also be used as an index, which can assist in more accurate detection of breast cancer.

Additionally, as specifically described in Examples below, the detection of breast cancer can also be assisted by measuring the abundance ratio of isoforms (isomiRs) of miR-150-5p (SEQ ID NO: 83) and/or miR-26b-5p (SEQ ID NO: 126) to the same microRNA(s) in the mature miRNA form contained in serum or plasma isolated from a living body (where "the abundance of isoforms (isomiRs)" refers to the total abundance of sequences in which 1 to 5 nucleotides are deleted from or added to the 3' or 5' end of a mature miRNA). In this case, a higher abundance ratio than that of healthy subjects indicates a higher likelihood of having breast cancer. This method shows a very high statistically significant difference (with a very small *p*-value) and is therefore considered as an accurate method.

Similarly, as specifically described in Examples below, the detection of breast cancer can also be assisted by measuring the abundance ratio of isoforms (isomiRs) of miR-93-5p (SEQ ID NO: 155) and/or miR-17-5p (SEQ ID NO: 282) to the same microRNA(s) in the mature miRNA form contained in serum or plasma isolated from a living body (where "the abundance of isoforms (isomiRs)" refers to the total abundance of sequences in which 1 to 5 nucleotides are deleted from or added to the 3' or 5' end of a mature miRNA). In this case, a lower abundance ratio than that of healthy subjects indicates a higher likelihood of having breast cancer. This method shows a very high statistically significant difference (with a very small *p*-value) and is therefore considered as an accurate method.

Moreover, a method of detecting the abundance of miRNAs or the like in a test sample from an individual suspected of having or affected with breast cancer is also provided.

That is, a method of detecting the abundance of at least one of miRNAs, isoform miRNAs (isomiRs), precursor miRNAs, transfer RNA fragments (tRFs), or non-coding RNA fragments (RRNAs, snoRNAs, or LincRNAs) whose nucleotide sequence is represented by any one of SEQ ID NOs: 2, 21, 22, 23, 24, 26, 30, 31, 32, 33, 34, 152, 151, 15, 28, 41, 1, 14, 27, 40. 25, 12, 160, 3 to 11, 13, 16 to 20, 29, 35 to 39, 42 to 150, 153 to 159, 161 to 265 in a test sample from an individual suspected of having or affected with breast cancer is also provided, wherein the method includes the steps of:
collecting a blood sample from the individual; and
measuring the abundance of the RNA strand(s) in the blood sample by means of a next-generation sequencer or qRT-PCR;
wherein the abundance of at least one of the miRNAs, isomiRs, precursor miRNAs, transfer RNA fragments, or non-coding RNA fragments whose nucleotide sequence is represented by any one of SEQ ID NOs: 1 to 19, 27, 28, 34 to 51, 74, 76, 77, 80 to 84, 96, 101 to 104, 115 to 122, 125, 128, 134 to 139, 151, 152, 159 to 165, 168, 169, 174, and 175 to 199 is higher in patients than in healthy subjects, or the abundance of at least one of the miRNAs, isomiRs, precursor miRNAs, transfer RNA fragments, or non-coding RNA fragments whose nucleotide sequence is represented by any one of SEQ ID NOs: 20 to 26, 29 to 33, 52 to 54, 56 to 73, 75, 78 to 79, 85 to 95, 97 to 100, 105 to 114, 123, 124. 126. 127. 129 to 133, 140 to 150, 153 to 158, 166, 167, 170 to 173, and 200 to 265 is lower in patients than in healthy subjects.

Additionally, in cases where the detection of breast cancer is successfully achieved by the above-described method of the present invention, an effective amount of an anti-breast cancer drug can be administered to patients in whom breast cancer is detected, to treat the breast cancer. Examples of the anti-breast cancer drug can include Herceptin, trastuzumab, pertuzumab, trastuzumab emtansine-paclitaxel, docetaxel, vinorelbine, lapatinib, and capecitabine.

The present invention will be specifically described below by way of examples and comparative examples. However, the present invention is not limited to the examples below.

### Examples 1 to 269 and Comparative Examples 1 to 12

### 1. Materials and Methods

### (1) Clinical Samples

Serum samples from 109 patients with breast cancer and 72 healthy subjects were used. The numbers of patients with breast cancer at stage 0 and at stage 1 or later were 6 and 134, respectively.

### (2) Extraction of RNA in Serum

Extraction of RNA in serum was performed using the miRNeasy Mini kit (QIAGEN).
1) Each frozen serum sample was thawed and centrifuged at 10,000 rpm for 5 minutes at room temperature to precipitate aggregated proteins and blood cell components.
2) To a new 1.5-mL tube, 200 µL of the supernatant was transferred.
3) To the tube. 1000 µL of the QIAzol Lysis Reagent was added and mixed thoroughly to denature protein components.
4) To the tube, 10 µL of 0.05 nM cel-miR-39 was added as a control RNA for RNA extraction, mixed by pipetting, and then left to stand at room temperature for 5 minutes.
5) To promote separation of the aqueous and organic solvent layers, 200 µL of chloroform was added to the tube, mixed thoroughly, and left to stand at room temperature for 3 minutes.
6) The tube was ccntrifuged at 12,000 x g for 15 minutes at 4°C and 650 µL of the upper aqueous layer was transferred to a new 2-mL tube.
7) For the separation of RNA, 975 µL of 100% ethanol was added to the tube and mixed by pipetting.
8) To a miRNeasy Mini spin column (hereinafter referred to as "column"), 650 µL of the mixture in the step 7 was transferred, left to stand at room temperature for 1 minute, and then centrifuged at 8000 x g for 15 seconds at room temperature to allow RNA to be adsorbed on the filter of the column. The flow-through solution from the column was discarded.
9) The step 8 was repeated until the total volume of the solution of the step 7 was filtered through the column to allow all the RNA to be adsorbed on the filter.
10) To remove impurities attached on the filter, 650 µL of Buffer RWT was added to the column and centrifuged at 8000 x g for 15 seconds at room temperature. The flow-through solution from the column was discarded.
11) To clean the RNA adsorbed on the filter, 500 µL of Buffer RPE was added to the column and centrifuged at 8000 x g for 15 seconds at room temperature. The flow-through solution from the column was discarded.
12) To clean the RNA adsorbed on the filter, 500 µL of Buffer RPE was added to the column and centrifuged at 8000 x g for 2 minutes at room temperature. The flow-through solution from the column was discarded.
13) To completely remove any solution attached on the filter, the column was placed in a new 2-mL collection tube and centrifuged at 10,000 x g for 1 minute at room temperature.
14) The column was placed into a 1.5-mL tube and 50 µL of RNase-free water was added thereto and left to stand at room temperature for 1 minute.
15) Centrifugation was performed at 8000 x g for 1 minute at room temperature to elute the RNA adsorbed on the filter. The eluted RNA was used in the following experiment without further purification and the remaining portion of the eluted RNA was stored at -80°C.

### (3) Extraction of RNA from Exosomes

Exosomes in serum were isolated with the Total Exosome Isolation (from serum), a commercially available kit from Thermo Fisher Scientific, Inc. Extraction of RNA from the collected exosomes was performed using the miRNeasy Mini kit (trade name, manufactured by QIAGEN).

### (4) Quantification of miRNAs or the Like

The quantification of miRNAs or the like was performed as follows. In cases where miRNAs or the like from, for example, two groups were quantified, extracellular vesicles (including exosomes) isolated by the same method were used to extract RNAs through the same method, from which cDNA libraries were prepared and then analyzed by next-generation sequencing. The next-generation sequencing analysis is not limited by a particular instrument, provided that the instrument determines sequences.

### (5) Calculation of Cut-off Value and AUC

Specifically, the cut-off value and the AUC were calculated from measurement results as follows. The logistic regression analysis was carried out using the JMP Genomics 8 (trade name) to draw the ROC curve and to calculate the AUC. Moreover, the value corresponding to a point on the ROC curve which was closest to the upper left corner of the ROC graph (sensitivity: 1.0, specificity: 1.0) was defined as the cut-off value.

### 2. Results

The results are presented in Table 2.

**Table 2-1**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 1 | 1 | isomiR | mir-7-1//mir-7-2//mir-7-3 5p | Mature 5' sub | 22 | 472 | 26 | 4.75 | 0.981 | 5.42 |
| Example 2 | 2 | isomiR | mir-15a 5p | Mature 5' sub | 21 | 122 | 1 | 5.67 | 1.000 | 3.85 |
| Comparative Example 1 | 270 | miRNA | mir-15a 5p | Mature 5' | 22 | 34 | 25 | 0 | | |
| Example 3 | 3 | isomiR | mir-181a-2//mir-181a-1 5p | Mature 5' sub | 22 | 6211 | 518 | 3.19 | 0.932 | 10.76 |
| Example 4 | 4 | isomiR | mir-181a-2//mir-181a-1 5p | Mature 5' sub | 21 | 9190 | 616 | 3.35 | 0.935 | 11.35 |
| Example 5 | 5 | isomiR | mir-181a-2//mir-181a-1 5p | Mature 5' sub | 20 | 8635 | 732 | 3.41 | 0.927 | 10.64 |
| Example 6 | 6 | isomiR | mir-181a-2//mir-181a-1 5p | Mature 5' sub | 19 | 5479 | 477 | 3.67 | 0.913 | 10.37 |
| Example 7 | 7 | isomiR | mir-181a-2//mir-181a-1 5p | Mature 5' sub | 18 | 9102 | 684 | 3.65 | 0.924 | 10.39 |
| Example 8 | 8 | precursor | mir-181a-2//mir-181a-1 5p | precursor miRNA | 17 | 7489 | 520 | 3.31 | 0.934 | 10.96 |
| Example 9 | 9 | precursor | mir-181a-2//mir-181a-1 5p | precursor miRNA | 16 | 4007 | 327 | 3.67 | 0.903 | 9.33 |
| Example 10 | 10 | precursor | mir-181a-2//mir-181a-1 5p | precursor miRNA | 15 | 3288 | 444 | 3.22 | 0.879 | 9.20 |
| Example 11 | 11 | precursor | mir-181a-2//mir-181a-1 5p | precursor miRNA | 15 | 103 | 7 | 3.24 | 0.876 | 4.40 |
| Example 12 | 12 | TRF | Homo_ sapiens_tRNA-Gly-CCC-1-1//···*1 | Exact | 32 | 1484 | 235 | 2.69 | 0.974 | 8.49 |
| Example 13 | 13 | tRF | Homo_ sapiens_tRNA-Gly-CCC-1-1//···*1 | Exact | 31 | 583 | 174 | 1.74 | 0.834 | 7.88 |
| Comparative Example2 | 271 | tRF | Homo_sapiens_tRNA-Gly-CCC-1-1//···*1 | Exact | 30 | 48049 | 49581 | -0.05 | | |
| Example 14 | 14 | tRF | Homo_sapiens_tRNA-Gly-CCC-2-1//Homo_s apiens_tRNA-Gly-CCC-2-2 | Exact | 32 | 69796 | 11854 | 2.87 | 0.977 | 15.05 |
| Example 15 | 15 | tRF | the same as above | Exact | 31 | 37144 | 6320 | 2.50 | 0.989 | 13.78 |
| Example 16 | 16 | miRNA | mir-423 3p | Mature 3' | 23 | 594 | 236 | 1.65 | 0.937 | 8.44 |
| Example 17 | 17 | tRF | Homo_ sapiens_tRNA-iMet-CAT-1-1//···*2 | Exact | 31 | 30897 | 12188 | 1.44 | 0.824 | 13.24 |
| Example 18 | 18 | miRNA | mir-4286 5p | Mature 5' | 17 | 5 | 1 | 1.41 | 0.739 | 1.68 |
| Example 19 | 19 | isomiR | mir-150 5p | Mature 5' sub | 21 | 560 | 221 | 1.35 | 0.792 | 8.48 |
| Example 20 | 20 | isomiR | mir-16-1//mir-16-2 5p | Mature 5' sub | 19 | 40 | 138 | -1.69 | 0.869 | 6.44 |
| Comparative Example3 | 272 | miRNA | mir-16-1//mir-16-2 5p | Mature 5' | 22 | 3556 | 3388 | 0.07 | | |
| Comparative Example4 | 273 | miRNA | let-7g 5p | Mature 5' | 22 | 261 | 196 | 0.41 | | |
| Example 21 | 21 | isomiR | let-7g 5p | Mature 5' sub | 21 | 1 | 503 | -8.56 | 1 | 4.71 |
| Example 22 | 22 | isomiR | let-7g 5p | Mature 5' sub | 20 | 0 | 339 | -8.09 | 1 | 1.68 |
| Example 23 | 23 | isomiR | let-7g 5p | Mature 5' sub | 19 | 0 | 301 | -7.93 | 1 | 0.8 |
| Example 24 | 24 | isomiR | let-7g 5p | Mature 5' sub | 18 | 0 | 277 | -7.78 | 1 | 3.15 |

**Table 2-2**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 25 | 25 | isomiR | let-7g 5p | Mature 5' sub | 17 | 0 | 120 | -6.24 | 0.975 | 0 |
| Example 26 | 26 | precursor | let-7g 5p | precursor miRNA | 15 | 0 | 112 | -6.38 | 1 | 3.05 |
| Example 27 | 27 | tRF | Homo_ sapiens_tRNA-Gly-CCC-2-1//Homo_s apiens_tRNA-Gly-CCC-2-2 | Exact | 32 | 69796 | 11854 | 2.87 | 0.977 | 15.05 |
| Example 28 | 28 | tRF | the same as above | Exact | 31 | 37144 | 6320 | 2.50 | 0.989 | 13.78 |
| Example 29 | 29 | miRNA | mir-101-1//mir-101-2 3p | Mature 3' | 21 | 41 | 116 | -1.86 | 0.861 | 6.07 |
| Example 30 | 30 | isomiR | mir-24-1//mir-24-2 3p | Mature 3' sub | 20 | 1 | 119 | -6.40 | 1.000 | 5.35 |
| Example 31 | 31 | precursor | mir-24-1//mir-24-2 3p | precursor miRNA | 16 | 0 | 81 | -5.66 | 1 | 2.4 |
| Example 32 | 32 | isomiR | mir-96 5p | Mature 5' sub | 22 | 0 | 316 | -7.93 | I | 2.22 |
| Example 33 | 33 | isomiR | mir-96 5p | Mature 5' sub | 21 | 0 | 273 | -7.79 | 1 | 2.69 |
| Comparative Example5 | 274 | miRNA | mir-96 5p | Mature 5' | 23 | 9 | 7 | 0.26 | | |
| Example 34 | 34 | tRF | Homo_sapiens_tRNA-Glu-CTC-1-1//···*3 | Exact | 35 | 30 | 14 | 1.03 | 0.71 | 5.13 |
| Example 35 | 35 | tRF | Homo_ sapiens_tRNA-Glu-CTC-1-1//···*3 | Exact | 32 | 5436 | 5209 | 0.29 | 0.61 | 11.56 |
| Comparative Example6 | 275 | tRF | Homo_sapiens_tRNA-Glu-CTC-1-1//···*3 | Exact | 31 | 448 | 337 | 0.41 | | |
| Example 36 | 36 | tRF | Homo_ sapiens_tRNA-Glu-CTC-1-1//···*3 | Exact | 30 | 220 | 196 | 0.26 | 0.59 | 7.01 |
| Example 37 | 37 | tRF | Homo_ sapiens_tRNA-Gly-GCC-1-1//···*4 | Exact | 32 | 74 | 15 | 2.66 | 0.899 | 4.45 |
| Comparative Example7 | 276 | tRF | Homo_sapiens_tRNA-Gly-GCC-1-1//···*4 | Exact | 30 | 1862 | 2434 | -0.39 | | |
| Example 38 | 38 | tRF | Homo_sapiens_tRNA-Val-AAC-1-1//···*5 | Exact | 33 | 316 | 60 | 2.11 | 0.884 | 6.68 |
| Example 39 | 39 | tRF | Homo_sapiens_tRNA-Val-AAC-1-1//···*5 | Exact | 32 | 281 | 104 | 1.58 | 0.755 | 8.03 |
| Comparative Example8 | 277 | tRF | Homo_sapiens_tRNA-Val-AAC-1-1//···*5 | Exact | 31 | 605 | 446 | 0.44 | | |
| Example 40 | 40 | tRF | Homo_sapiens_tRNA-Gly-CCC-2-1//Homo_s apiens_tRNA-Gly-CCC-2-2 | Exact | 32 | 69796 | 11854 | 2.87 | 0.977 | 15.05 |
| Example 41 | 41 | tRF | the same as above | Exact | 31 | 37144 | 6320 | 2.50 | 0.989 | 13.78 |
| Comparative Example9 | 278 | isomiR | mir-145 5p | Mature 5' sub | 22 | 506 | 494 | 0.04 | | |
| Example 42 | 42 | miRNA | mir-145 5p | Mature 5' | 23 | 92 | 38 | 1.39 | 0.815 | 5.64 |
| Example 43 | 43 | tRF | Homo_sapiens_tRNA-Val-TAC-1-1//Homo_sa piens_tRNA-Val-TAC-1-2 | Exact | 33 | 67 | 14 | 2.14 | 0.863 | 5.10 |
| Example 44 | 44 | tRF | Homo_ sapiens_tRNA-Lys-CTT-2-1//···*6 | Exact | 34 | 490 | 693 | 0.09 | 0.524 | 8.23 |
| Example 45 | 45 | tRF | Homo_sapiens_tRNA-Val-CAC-3-1 | Exact | 33 | 72 | 29 | 1.63 | 0.772 | 5.20 |

**Table 2-3**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 46 | 46 | isomiR | mir-21 5p | mature 5' super | 24 | 22 | 7 | 1.27 | 0.713 | 4.11 |
| Comparative Example 10 | 279 | miRNA | mir-21 5p | Mature 5' | 22 | 1793 | 1407 | 0.35 | | |
| Example 47 | 47 | tRF | Homo_ sapiens_tRNA-Lys-CTT-1-1//...*7 | Exact | 33 | 67.2 | 24.1 | 1.71 | 0.790 | 5.31 |
| Example 48 | 48 | tRF | Homo_ sapiens tRNA-Gly-CCC-1-1//...*8 | Exact | 32 | 1483.9 | 235.2 | 2.69 | 0.974 | 8.49 |
| Example 49 | 49 | tRF | Homo_sapiens_tRNA-Gly-CCC-1-1//...*8 | Exact | 31 | 583.4 | 174.0 | 1.74 | 0.834 | 7.88 |
| Comparative Example 1 | 280 | tRF | Homo_sapiens_tRNA-Gly-CCC-1-1//...*8 | Exact | 30 | 43906 | 46524 | -0.08 | | |
| Example 50 | 50 | tRF | Homo_ sapiens_tRNA-Ala-AGC-4-1//...*9 | Exact | 32 | 895 | 159 | 2.33 | 0.955 | 8.46 |
| Example 51 | 51 | tRF | Homo_sapiens tRNA-Lys-CTT-1-1//...*10 | Exact | 33 | 67 | 24 | 1.71 | 0.790 | 5.31 |
| Example 52 | 52 | tRF | Homo_sapiens_tRl\A-Gly-GCC-1-1//...*11 | Exact | 32 | 74 | 15 | 2.66 | 0.899 | 4.45 |
| Example 53 | 53 | isomiR | mir-10a 5p | Mature 5' sub | 21 | 22 | 51 | -0.66 | 0.576 | 5.62 |
| Example 54 | 54 | isomiR | mir-22 3p | Mature 3' sub | 19 | 31 | 87 | -0.46 | 0.558 | 6.63 |
| Comparative Example 12 | 281 | miRNA | mir-22 3p | Mature 3' | 22 | 1773 | 1849 | None | | |
| Example 55 | 55 | miRNA | mir-16-2 3p | Mature 3' | 22 | 0 | I | -0.14 | 0.519 | 1.07 |
| Example 56 | 56 | isomiR | let-7a-1//let-7a-2//let-7a-3 5p | Mature 5' sub | 20 | 441 | 1147 | -1.62 | 0.854 | 9.66 |
| Example 57 | 57 | isomiR | let-7b 5p | Mature 5' sub | 20 | 285 | 521 | -0.84 | 0.806 | 8.62 |
| Example 58 | 58 | miRNA | let-7b 5p | Mature 5' | 22 | 796 | 1591 | -1.20 | 0.826 | 10.21 |
| Example 59 | 59 | isomiR | let-7b 5p | Mature 5' sub | 21 | 542 | 1094 | -0.95 | 0.823 | 9.52 |
| Example 60 | 60 | isomiR | let-7f-1//let-7f-2 5p | Mature 5' sub | 20 | 169 | 477 | -2.11 | 0.879 | 7.96 |
| Example 61 | 61 | isomiR | let-7g 5p | Mature 5' sub | 21 | 661 | 1212 | -0.78 | 0.777 | 9.26 |
| Example 62 | 62 | isomiR | tet-7g 5p | Mature 5' sub | 20 | 127 | 265 | -1.24 | 0.834 | 7.44 |
| Example 63 | 63 | isomiR | let-7i 5p | Mature 5' sub | 21 | 645 | 1289 | -1.17 | 0.885 | 9.92 |
| Example 64 | 64 | isomiR | let-7i 5p | Mature 5' sub | 20 | 179 | 338 | -0.80 | 0.831 | 8.06 |
| Example 65 | 65 | isomiR | mir-101-1//mir-101-2 3p | Mature 3' sub/super | 21 | 485 | 825 | -0.53 | 0.824 | 9.38 |
| Example 66 | 66 | isomiR | mir-101-1//mir-101-2 3p | Mature 3' super | 22 | 571 | 1203 | -1.51 | 0.861 | 9.88 |
| Example 67 | 67 | isomiR | mir-101-1//mir-101-2 3p | Mature 3' sub | 20 | 105 | 213 | -1.36 | 0.812 | 7 |
| Example 68 | 68 | isomiR | mir-103a-2//mir-103a-1//mir-107 3p | Mature 3' sub | 20 | 390 | 764 | -0.67 | 0.85 | 9.1 |
| Example 69 | 69 | isomiR | mir-103a-2//mir-103a-1//mir-107 3p | Mature 3' sub | 19 | 264 | 473 | -1.16 | 0.800 | 8.18 |

**Table 2-4**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 70 | 70 | isomiR | mir-103a-2//mir-103a-1//mir-107 3p | Mature 3' sub | 21 | 911 | 1932 | -1.22 | 0.926 | 10.63 |
| Example 71 | 71 | miRNA | mir-106a 5p | Mature 5' | 23 | 336 | 537 | -0.81 | 0.824 | 8.62 |
| Example 72 | 72 | isomiR | mir-106b 5p | Mature 5' sub | 20 | 304 | 667 | -0.90 | 0.894 | 9.15 |
| Example 73 | 73 | miRNA | mir-106b 5p | Mature 5' | 21 | 277 | 585 | -0.87 | 0.824 | 8.58 |
| Example 74 | 74 | miRNA | mir-130a 3p | Mature 3' | 22 | 495 | 299 | 0.90 | 0.673 | 8.72 |
| Example 75 | 75 | isomiR | mir-130a 3p | Mature 3' sub | 21 | 63 | 91 | -0.79 | 0.724 | 5.77 |
| Example 76 | 76 | isomiR | mir-140 3p | Mature 3' sub/super | 22 | 399 | 323 | 0.59 | 0.643 | 8.49 |
| Example 77 | 77 | isomiR | mir-140 3p | Mature 3' sub/super | 23 | 189 | 177 | 0.29 | 0.547 | 7.57 |
| Example 78 | 78 | isomiR | mir-142 5p | Mature 5' sub/super | 20 | 213 | 478 | -0.99 | 0.879 | 8.36 |
| Example 79 | 79 | isomiR | mir-144 3p | Mature 3' sub | 19 | 168 | 481 | -1.28 | 0.905 | 8.04 |
| Example 80 | 80 | isomiR | mir-145 5p | Mature 5' sub | 22 | 452 | 335 | 0.70 | 0.635 | 8.73 |
| Example 81 | 81 | isomiR | mir-146a 5p | Mature 5' sub | 21 | 169 | 90 | 1.21 | 0.774 | 6.13 |
| Example 82 | 82 | miRNA | mir-146a 5p | Mature 5' | 22 | 715 | 358 | 1.51 | 0.819 | 8.57 |
| Example 83 | 83 | miRNA | mir-150 5p | Mature 5' | 22 | 1298 | 597 | 0.88 | 0.752 | 10.25 |
| Example 84 | 84 | miRNA | mir-151a 5p | Mature 5' | 21 | 294 | 225 | 0.61 | 0.597 | 7.5 |
| Example 85 | 85 | isomiR | mir-15a 5p | Mature 5' sub | 21 | 1780 | 3900 | -0.94 | 0.884 | 11.26 |
| Example 86 | 86 | isomiR | mir-15a 5p | Mature 5' sub | 20 | 347 | 1111 | -2.03 | 0.910 | 9.44 |
| Example 87 | 87 | isomiR | mir-15b 5p | Mature 5' sub | 20 | 163 | 383 | -1.47 | 0.781 | 7.43 |
| Example 88 | 88 | isomiR | mir-15b 5p | Mature 5' sub | 19 | 146 | 290 | -0.97 | 0.803 | 7.37 |
| Example 89 | 89 | isomiR | mir-16-1//mir-16-2 5p | Mature 5' super | 23 | 553 | 991 | -0.78 | 0.758 | 9.14 |
| Example 90 | 90 | isomiR | mir-16-1//mir-16-2 5p | Mature 5' sub | 20 | 1306 | 3416 | -1.87 | 0.900 | 11.01 |
| Example 91 | 91 | isomiR | mir-16-1//mir-16-2 5p | Mature 5' sub | 21 | 495 | 1339 | -1.80 | 0.919 | 9.45 |
| Example 92 | 92 | isomiR | mir-16-2 3p | Mature 3' sub/super | 20 | 54 | 162 | -1.84 | 0.886 | 6.80 |
| Example 93 | 93 | isomiR | mir-17 5p | Mature 5' sub | 20 | 98 | 215 | -1.13 | 0.872 | 7.12 |
| Example 94 | 94 | isomiR | mir-17 5p | Mature 5' sub | 21 | 1036 | 2583 | -1.44 | 0.902 | 10.58 |

**Table 2-5**

| Example | S EQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 95 | 95 | isomiR | mir-17//mir-106a 5p | Mature 5' sub | 22 | 93 | 123 | -0.57 | 0.689 | 6.51 |
| Example 96 | 96 | miRNA | mir-181a-2//mir-181a-1 5p | Mature 5' | 23 | 266 | 130 | 1.26 | 0.818 | 8.02 |
| Example 97 | 97 | miRNA | mir-18a 5p | Mature 5' | 23 | 372 | 552 | -0.27 | 0.713 | 8.98 |
| Example 98 | 98 | isomiR | mir-18a 5p | Mature 5' sub | 22 | 164 | 325 | -0.80 | 0.8 | 7.63 |
| Example 99 | 99 | isomiR | mir-18a 5p | Mature 5' sub | 21 | 56 | 100 | -1.00 | 0.661 | 6.27 |
| Example 100 | 100 | isomiR | mir-18a 5p | Mature 5' sub | 20 | 113 | 315 | -1.47 | 0.915 | 7.43 |
| Example 101 | 101 | isomiR | mir-191 5p | Mature 5' super | 24 | 475 | 272 | 1.05 | 0.731 | 7.96 |
| Example 102 | 102 | isomiR | mir-191 5p | Mature 5' sub | 22 | 944 | 595 | 0.69 | 0.779 | 9.37 |
| Example 103 | 103 | miRNA | mir-193a 5p | Mature 5' | 22 | 615 | 368 | 1.44 | 0.821 | 8.24 |
| Example 104 | 104 | isomiR | mir-197 3p | Mature 3' sub | 21 | 142 | 84 | 0.73 | 0.728 | 7 |
| Example 105 | 105 | miRNA | mir-19a 3p | Mature 3' | 23 | 1644 | 3964 | -1.36 | 0.960 | 11.61 |
| Example 106 | 106 | isomiR | mir-19a 3p | Mature 3' sub | 22 | 844 | 2171 | -1.40 | 0.952 | 10.50 |
| Example 107 | 107 | isomiR | mir-19a 3p | Mature 3' sub | 21 | 545 | 1667 | -1.66 | 0.952 | 9.87 |
| Example 108 | 108 | isomiR | mir-19b-1//mir-19b-2 3p | Mature 3' sub | 20 | 99 | 259 | -1.22 | 0.913 | 7.32 |
| Example 109 | 109 | isomiR | mir-19b-1//mir-19b-2 3p | Mature 3' sub | 21 | 3597 | 7074 | -1.05 | 0.903 | 12.46 |
| Example 110 | 110 | miRNA | mir-20a 5p | Mature 5' | 23 | 1499 | 3378 | -1.42 | 0.845 | 10.98 |
| Example 111 | 111 | isomiR | mir-20a 5p | Mature 5' sub | 22 | 153 | 391 | -1.64 | 0.818 | 7.37 |
| Example 112 | 112 | isomiR | mir-20a 5p | Mature 5' sub | 21 | 515 | 1427 | -1.72 | 0.898 | 9.57 |
| Example 113 | 113 | miRNA | mir-20b 5p | Mature 5' | 23 | 255 | 554 | -0.93 | 0.885 | 8.54 |
| Example 114 | 114 | isomiR | mir-20b 5p | Mature 5' sub | 21 | 88 | 217 | -1.32 | 0.866 | 7.13 |
| Example 115 | 115 | isomiR | mir-223 3p | Mature 3' sub | 21 | 2137 | 1020 | 1.02 | 0.856 | 10.73 |
| Example 116 | 116 | isomiR | mir-223 3p | Mature 3' sub/super | 22 | 4722 | 2726 | 0.68 | 0.756 | 11.66 |
| Example 117 | 117 | isomiR | mir-223 3p | Mature 3' sub | 20 | 429 | 301 | 0.49 | 0.663 | 8.17 |
| Example 118 | 118 | isomiR | mir-223 3p | Mature 3' sub | 21 | 1217 | 827 | 0.49 | 0.682 | 9.84 |
| Example 119 | 119 | isomiR | mir-223 3p | Mature 3' super | 23 | 14613 | 10018 | 0.45 | 0.648 | 14.11 |
| Example 120 | 120 | miRNA | mir-223 3p | Mature 3' | 22 | 11070 | 7880 | 0.42 | 0.627 | 13.66 |
| Example 121 | 121 | isomiR | mir-24-1//mir-24-2 3p | Mature 3' sub | 19 | 230 | 167 | 0.86 | 0.698 | 7.7 |

**Table 2-6**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 122 | 122 | miRNA | mir-24-1//mir-24-2 3p | Mature 3' | 22 | 354 | 211 | 0.80 | 0.695 | 8.5 |
| Example 123 | 123 | isomiR | mir-25 3p | Mature 3' sub | 20 | 119 | 239 | -0.83 | 0.877 | 7.55 |
| Example 124 | 124 | isomiR | mir-25 3p | Mature 3' sub | 21 | 260 | 537 | -1.03 | 0.881 | 8.58 |
| Example 125 | 125 | miRNA | mir-26a-1//mir-26a-2 5p | Mature 5' | 22 | 540 | 391 | 0.17 | 0.581 | 9.06 |
| Example 126 | 126 | miRNA | mir-26b 5p | Mature 5' | 21 | 267 | 629 | -2.02 | 0.856 | 8.60 |
| Example 127 | 127 | isomiR | mir-26b 5p | Mature 5' sub | 20 | 39 | 112 | -2.39 | 0.895 | 6.34 |
| Example 128 | 128 | miRNA | mir-29a 3p | Mature 3' | 22 | 91 | 69 | 0.54 | 0.968 | 7.01 |
| Example 129 | 129 | miRNA | mir-29c 3p | Mature 3' | 22 | 11 | 21 | -1.39 | 0.758 | 3.59 |
| Example 130 | 130 | isomiR | mir-29c 3p | Mature 3' sub | 21 | 26 | 61 | -1.68 | 0.794 | 5.31 |
| Example 131 | 131 | isomiR | mir-30d 5p | Mature 5' sub | 20 | 169 | 272 | -0.66 | 0.778 | 7.83 |
| Example 1 32 | 132 | isomiR | mir-30e 5p | Mature 5' sub/super | 23 | 345 | 603 | -1.13 | 0.874 | 8.94 |
| Example 133 | 133 | isomiR | mir-30e 5p | Mature 5' super | 24 | 550 | 1043 | -1.11 | 0.861 | 9.84 |
| Example 134 | 134 | isomiR | mir-320a 3p | Mature 3' sub/super | 22 | 190 | 156 | 0.91 | 0.755 | 6.29 |
| Example 135 | 135 | isomiR | mir-342 3p | Mature 3' sub | 22 | 485 | 267 | 0.69 | 0.678 | 8.58 |
| Example 136 | 136 | miRNA | mir-342 3p | Mature 3' | 23 | 235 | 170 | 0.44 | 0.625 | 7.66 |
| Example 137 | 137 | isomiR | mir-34a 5p | Mature 5' sub | 20 | 218 | 68 | 2.07 | 0.905 | 6.13 |
| Example 138 | 138 | isomiR | mir-423 5p | Mature 5' sub | 19 | 189 | 162 | 0.58 | 0.635 | 6.49 |
| Example 139 | 139 | miRNA | mir-423 5p | Mature 5' | 23 | 1108 | 690 | 0.76 | 0.795 | 9.19 |
| Example 140 | 140 | miRNA | mir-425 5p | Mature 5' | 23 | 601 | 1036 | -0.44 | 0.921 | 9.66 |
| Example 141 | 141 | isomiR | mir-451a 5p | Mature 5' sub | 21 | 210 | 410 | -1.06 | 0.834 | 7.87 |
| Example 142 | 142 | isomiR | mir-451a 5p | Mature 5' sub | 20 | 10526 | 22072 | -1.18 | 0.850 | 14.18 |
| Example 143 | 143 | isomiR | mir-451a 5p | Mature 5' super | 25 | 15882 | 35342 | -1.22 | 0.871 | 14.35 |
| Example 144 | 144 | isomiR | mir-451a 5p | Mature 5' super | 24 | 1781 | 3852 | -1.27 | 0.837 | 10.84 |
| Example 145 | 145 | isomiR | mir-451a 5p | Mature 5' sub | 17 | 41 | 102 | -1.45 | 0.892 | 6.23 |

**Table 2-7**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 146 | 146 | isomiR | mir-451a 5p | Mature 5' super | 23 | 40452 | 93174 | -1.30 | 0.923 | 16.10 |
| Example 147 | 147 | isomiR | mir-451a 5p | Mature 5' sub | 19 | 397 | 908 | -1.11 | 0.861 | 9.26 |
| Example 148 | 148 | isomiR | mir-451a 5p | Mature 5' sub | 21 | 25677 | 61536 | -1.34 | 0.895 | 15.18 |
| Example 149 | 149 | miRNA | mir-451a 5p | Mature 5' | 22 | 84474 | 211366 | -1.44 | 0.919 | 17.37 |
| Example 150 | 150 | isomiR | mir-486-1//mir-486-2 5p | Mature 5' sub | 20 | 329 | 337 | 0.21 | 0.432 | 8.91 |
| Example 151 | 151 | isomiR | mir-7-1//mir-7-2//mir-7-3 5p | Mature 5' sub | 21 | 299 | 8 | 5.47 | 0.998 | 5.78 |
| Example 152 | 152 | isomiR | mir-7-1//mir-7-2//mir-7-3 5p | Mature 5' sub | 20 | 393 | 22 | 4.46 | 1.000 | 6.33 |
| Example 153 | 153 | miRNA | mir-92a-1//mir-92a_2 3p | Mature 3' | 22 | 2039 | 3491 | -0.91 | 0.771 | 11.33 |
| Example 154 | 154 | isomiR | mir-92a-1//mir-92a-2 3p | Mature 3' sub | 21 | 225 | 391 | -0.68 | 0.800 | 8.19 |
| Example 155 | 155 | miRNA | mir-93 5p | Mature 5' | 23 | 2376 | 4073 | -0.44 | 0.824 | 11.76 |
| Example 156 | 156 | isomiR | mir-93 5p | Mature 5' sub | 20 | 45 | 102 | -1.25 | 0.879 | 6.05 |
| Example 157 | 157 | isomiR | mir-93 5p | Mature 5' sub | 21 | 355 | 958 | -1.20 | 0.911 | 9.11 |
| Example 158 | 158 | tRF | Homo_ sapiens_tRNA-Ala-AGC-2-1//...*12 | Exact | 30 | 125 | 386 | -1.74 | 0.885 | 7.62 |
| Example 159 | 159 | tRF | Homo_ sapiens_tRNA-Glu-CTC-1-1//...*3 | Exact | 26 | 855 | 463 | 0.78 | 0.792 | 9.51 |
| Example 160 | 160 | tRF | Homo_sapiens_ tRNA-Gly-CCC-2-1//-*13 | Exact | 31 | 571 | 53 | 3.77 | 0.971 | 7.13 |
| Example 161 | 161 | tRF | Homo_sapiens_ tRNA-Gly-CCC-2-1//...*13 | Exact | 29 | 533 | 85 | 2.33 | 0.923 | 7.00 |
| Example 162 | 162 | tRF | Homo_sapiens tRNA-Gly-CCC-2-1//...*13 | Exact | 30 | 473 | 48 | 3.29 | 0.968 | 7.07 |
| Example 163 | 163 | tRF | Homo_ sapiens_tRNA-Gly-CCC-2-1//...*13 | Exact | 30 | 60319 | 23392 | 1.25 | 0.882 | 14.71 |
| Example 164 | 164 | tRF | Homo_sapiens_tRNA-Gly-CCC-2-1//...*13 | Exact | 26 | 361 | 177 | 1.29 | 0.932 | 8.00 |
| Example 165 | 165 | tRF | Homo_sapiens_ tRNA-Gly-CCC-2-//...*13 | Exact | 22 | 1121 | 673 | 0.74 | 0.845 | 9.75 |
| Example 166 | 166 | tRF | Homo_sapiens_ tRNA-Gly-CCC-2-1//...*13 | Exact | 27 | 1197 | 2420 | -1.03 | 0.882 | 10.79 |
| Example 167 | 167 | tRF | Homo_sapiens_tRNA-Gly-GCC-1-1//...*4 | Exact | 25 | 255 | 762 | -1.62 | 0.878 | 8.42 |
| Example 168 | 168 | tRF | Homo_sapiens_tRNA-iMet-CAT-1-1//...*2 | Exact | 30 | 12545 | 8366 | 0.48 | 0.721 | 13 |
| Example 169 | 169 | tRF | Homo_sapiens_tRNA-iMet-CAT-1-1//...*2 | Exact | 29 | 4717 | 3743 | 0.14 | 0.515 | 12.31 |
| Example 170 | 170 | tRF | Homo_ sapiens_tRNA-Lys-CTT-1-1//...*7 | Exact | 31 | 214 | 469 | -0.83 | 0.792 | 7.94 |
| Example 171 | 171 | tRF | Homo_sapiens_tRNA-Lys-CTT-1-1//...*7 | Exact | 32 | 1013 | 2634 | -1.19 | 0.700 | 9.60 |
| Example 172 | 172 | tRF | Homo_ sapiens_tRNA-Val-AAC-1-1//...*5 | Exact | 31 | 597 | 1844 | -1.30 | 0.829 | 9.7 |
| Example 173 | 173 | tRF | tRNA-Vat-CAC-3-1 ...*14 | Exact | 31 | 152 | 580 | -2.29 | 0.898 | 8.38 |

**Table 2-8**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 174 | 174 | tRF | Homo_ sapiens_tRNA-Gly-CCC-1-1//...*8 | Exact | 31 | 36471 | 45031 | -0.48 | 0.634 | 14.71 |
| Example 175 | 175 | LincRNA | ENST00000229465.10//...*24 | Exact | 17 | 2 | 29 | 3.79 | 0.930 | 3.48 |
| Example 176 | 176 | LincRNA | ENST00000229465.10//...*15 | Exact | 15 | 63 | 6 | 3.68 | 0.930 | 3.88 |
| Example 177 | 177 | RRNA | ENST00000616292.1//...*17 17 | Exact | 17 | 439 | 86 | 3.07 | 0.880 | 6.32 |
| Example 178 | 178 | tRF | Homo_ sapiens_tRNA-Gly-CCC-2-1//...*13 | Exact | 29 | 8 | 49 | 2.58 | 0.814 | 4.16 |
| Example 179 | 179 | snoRNA | ENST00000580533.1//...*25 | Exact | 23 | 6 | 33 | 2.52 | 0.879 | 3.64 |
| Example 180 | 180 | RRNA | ENST00000616292.1//...*19 | Exact | 16 | 105 | 21 | 2.44 | 0.867 | 4.99 |
| Example 181 | 181 | snoRNA | ENST00000580533.1//...*16 | Exact | 28 | 52 | 10 | 2.21 | 0.906 | 4.32 |
| Example 182 | 182 | snoRNA | ENST00000580533.11//...*26 | Exact | 27 | 17 | 69 | 2.02 | 0.870 | 5.25 |
| Example 183 | 183 | isomiR | mir-145 | Mature 5' sub | 18 | 151 | 35 | 1.90 | 0.869 | 6.30 |
| Example 184 | 184 | isomiR | mir-223 | Mature 3' sub | 19 | 212 | 83 | 1.90 | 0.792 | 5.85 |
| Example 185 | 185 | precursor | mir-145 | Precursor | 17 | 21 | 4 | 1.84 | 0.804 | 3.30 |
| Example 186 | 186 | isomiR | mir-23a//mir-23b | Mature 3' sub | 17 | 49 | 11 | 1.83 | 0.798 | 4.02 |
| Example 187 | 187 | tRF | Homo_sapiens_tRNA-Gly-CCC-2-1//...*13 | Exact | 24 | 54 | 28 | 1.82 | 0.744 | 3.89 |
| Example 188 | 188 | isomiR | mir-122 | Mature 5' sub | 19 | 68 | 20 | 1.82 | 0.746 | 3.82 |
| Example 189 | 189 | isomiR | mir-27a//mir-27b | Mature 3' sub | 18 | 35 | 10 | 1.75 | 0.803 | 4.17 |
| Example 190 | 190 | isomiR | mir-145 | Mature 5' sub | 20 | 76 | 28 | 1.75 | 0.812 | 5.14 |
| Example 191 | 191 | RRNA | ENST00000616292.1//...*21 | Exact | 15 | 48 | 15 | 1.70 | 0.789 | 4.29 |
| Example 192 | 192 | isomiR | mir-99a | Mature 5' sub | 21 | 50 | 21 | 1.67 | 0.735 | 4.09 |
| Example 193 | 193 | isomiR | mir-142 | Mature 3' sub | 22 | 130 | 38 | 1.67 | 0.864 | 6.09 |
| Example 194 | 194 | tRF | Homo_sapiens_tRNA-Gly-CCC-2-1//...*13 | Exact | 27 | 34 | 16 | 1.67 | 0.736 | 4.16 |
| Example 195 | 195 | isomiR | mir-145 | Mature 5' sub | 19 | 834 | 253 | 1.58 | 0.835 | 8.20 |
| Example 196 | 196 | isomiR | mir-122 | Mature 5' sub | 20 | 146 | 48 | 1.58 | 0.747 | 5.46 |
| Example 197 | 197 | isomiR | mir-30a | Mature 5' sub | 21 | 83 | 29 | 1.57 | 0.793 | 5.78 |
| Example 198 | 198 | isomiR | mir-27b | Mature 3' sub | 20 | 118 | 40 | 1.53 | 0.850 | 6.09 |
| Example 199 | 199 | isomiR | mir-23b | Mature 3' super | 22 | 93 | 33 | 1.51 | 0.867 | 5.51 |
| Example 200 | 200 | tRF | Homo_ sapiens_ tRNA-Ser-AGA-1-1//...*33 | Exact | 25 | 244 | 1260 | -1.51 | 0.773 | 8.66 |
| Example 201 | 201 | MiscRNA | ENST00000363745.1//...*23 | Exact | 23 | 18 | 54 | -1.53 | 0.762 | 4.40 |
| Example 202 | 202 | tRF | Homo_ sapiens_tRNA-Val-AAC-1-1//...*5 | Exact | 18 | 26 | 67 | -1.53 | 0.813 | 5.04 |

**Table 2-9**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 203 | 203 | tRF | Homo_sapiens_tRNA-Glu-TTC-2-1//...*31 | Exact | 26 | 18 | 47 | -1.54 | 0.808 | 5.09 |
| Example 204 | 204 | tRF | Homo_ sapiens_tRNA-Pro-AGG-1-1//...*34 | Exact | 25 | 55 | 152 | -1.55 | 0.790 | 6.30 |
| Example 205 | 205 | isomiR | mir-451a | Mature 5' sub | 19 | 19 | 39 | -1.56 | 0.733 | 4.03 |
| Example 206 | 206 | MiscRNA | ENST00000363745.1//...*22 | Exact | 24 | 15 | 45 | -1.56 | 0.737 | 4.77 |
| Example 207 | 207 | isomiR | mir-106a | Mature 5' sub | 22 | 43 | 106 | -1.57 | 0.766 | 5.64 |
| Example 208 | 208 | miRNA | mir-652 | Mature 3' | 21 | 12 | 27 | -1.58 | 0.751 | 3.84 |
| Example 209 | 209 | tRF | Homo_ sapiens_tRNA-Val-CAC-3-1 | Exact | 32 | 28 | 104 | -1.61 | 0.715 | 5.67 |
| Example 210 | 210 | tRF | Homo_ sapiens_tRNA-Glu-CTC-1-1//...*3 | Exact | 23 | 278 | 856 | -1.63 | 0.860 | 8.64 |
| Example 211 | 211 | tRF | Homo_sapiens_tRNA-Lys-CTT-1-1//...*10 | Exact | 28 | 6 | 18 | -1.65 | 0.777 | 3.43 |
| Example 212 | 212 | isomiR | mir-103a-2//mir-103a-1 | Mature 3' sub | 22 | 78 | 213 | -1.66 | 0.818 | 6.34 |
| Example 213 | 213 | tRF | Homo_sapiens_tRNA-Gly-CC-1-1//...*4 | Exact | 24 | 20 | 45 | -1.66 | 0.848 | 4.70 |
| Example 214 | 214 | tRF | Homo_sapiens_tRNA-Lys-CTT-2-1//...*6 | Exact | 30 | 20 | 45 | -1.66 | 0.772 | 3.84 |
| Example 215 | 215 | miRNA | mir-454 | Mature 3' | 23 | 12 | 34 | -1.67 | 0.771 | 4.73 |
| Example 216 | 216 | isomiR | mir-486-1//mir-486-2 | Mature 5' sub | 18 | 20 | 65 | -1.69 | 0.842 | 5.35 |
| Example 217 | 217 | tRF | Homo_sapiens_tR\A-Gly-GCC-1-1//...*11 | Exact | 23 | 17 | 44 | -1.69 | 0.889 | 4.74 |
| Example 218 | 218 | isomiR | mir-550a-1//mir-550a-2//mir-550a-3 | Mature 3' sub | 21 | 19 | 46 | -1.69 | . 0.803 | 4.22 |
| Example 219 | 219 | precursor | mir-486-1//mir-486-2 | Precursor | 16 | 30 | 86 | -1.69 | 0.817 | 6.05 |
| Example 220 | 220 | isomiR | mir-93 | Mature 5' sub | 22 | 55 | 155 | -1.70 | 0.820 | 6.42 |
| Example 221 | 221 | tRF | Homo_ sapiens_tRNA-Val-CAC-3-1 | Exact | 23 | 4 | 12 | -1.70 | 0.771 | 2.11 |
| Example 222 | 222 | tRF | Homo sapiens tRNA-Pro-AGG-1-1//...*34 | Exact | 27 | 17 | 54 | -1.70 | 0.774 | 4.53 |
| Example 223 | 223 | tRF | Homo_ sapiens_tRNA-Glu-TTC-2-1//...*31 | Exact | 28 | 8 | 26 | -1.71 | 0.761 | 4.50 |
| Example 224 | 224 | tRF | Homo_ sapiens_tRNA-Ser-GCT-1-1//...*32 | Exact | 25 | 253 | 1617 | -1.72 | 0.831 | 8.48 |
| Example 225 | 225 | isomiR | mir-451a | Mature 5' sub/super | 24 | 27 | 63 | -1.73 | 0.792 | 4.86 |
| Example 226 | 226 | tRF | Homo_ sapiens_tRNA-Ser-GCT-1-1//...*32 | Exact | 24 | 259 | 1271 | -1.76 | 0.848 | 8.73 |
| Example 227 | 227 | isomiR | mir-7-1//mir-7-2//mir-7-3 | Mature 5' sub | 22 | 12 | 31 | -1.76 | 0.761 | 4.19 |
| Example 228 | 228 | tRF | Homo_sapiens_tRNA-Cys-GCA-2-1//...*30 | Exact | 32 | 97 | 407 | -1.78 | 0.735 | 6.29 |
| Example 229 | 229 | tRF | Homo_sapiens_tRNA-Ser-AGA-1-1//...*33 | Exact | 23 | 37 | 183 | -1.79 | 0.757 | 5.49 |
| Example 230 | 230 | tRF | Homo_sapiens_tRNA-SeC-TCA-1-1 | Exact | 34 | 18 | 94 | -1.79 | 0.714 | 4.35 |
| Example 231 | 231 | tRF | Homo_ sapiens_tRNA-Ser-AGA-1-1/...*33 | Exact | 24 | 860 | 4453 | -1.80 | 0.785 | 10.42 |

**Table 2-10**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 232 | 232 | isomiR | mir-20b | Mature 5' sub | 22 | 18 | 44 | -1.83 | 0.770 | 4.78 |
| Example 233 | 233 | MiscRNA | ENST00000364228.1//···*18 | Exact | 23 | 45 | 132 | -1.85 | 0.910 | 5.95 |
| Example 234 | 234 | isomiR | mir-106b | Mature 3' sub/super | 22 | 8 | 22 | -1.90 | 0.793 | 3.31 |
| Example 235 | 235 | tRF | Homo sapiens tRNA-Glu-CTC-1-1//...*3 | Exact | 27 | 14 | 47 | -1.90 | 0.810 | 4.99 |
| Example 236 | 236 | tRF | Homo sapiens tRNA-Val-TAC-1-1//...*28 | Exact | 23 | 12 | 43 | -1.90 | 0.782 | 3.78 |
| Example 237 | 237 | tRF | Homo sapiens tRNA-Glu-CTC-1-1//...*3 | Exact | 24 | 50 | 183 | -1.93 | 0.850 | 6.68 |
| Example 238 | 238 | tRF | Homo sapiens tRNA-Ala-AGC-8-1//...*27 | Exact | 23 | 5 | 22 | -1.97 | 0.808 | 3.72 |
| Example 239 | 239 | MiscRNA | ENST00000363667.1//...*20 | Exact | 18 | 12 | 26 | -1.98 | 0.781 | 3.74 |
| Example 240 | 240 | tRF | Homo sapiens tRNA-Glu-TTC-2-1//...*31 | Exact | 29 | 18 | 52 | -1.98 | 0.832 | 4.86 |
| Example 241 | 241 | tRF | Homo sapiens tRNA-Val-CAG-2-1 | Exact | 23 | 4 | 19 | -2.04 | 0.813 | 3.60 |
| Example 242 | 242 | tRF | Homo sapiens tRNA-SeC-TCA-1-1 | Exact | 29 | 6 | 30 | -2.05 | 0.785 | 3.96 |
| Example 243 | 243 | tRF | Homo sapiens tRNA-Cys-GCA-2-1//...*30 | Exact | 31 | 12 | 72 | -2.08 | 0.757 | 4.67 |
| Example 244 | 244 | isomiR | mir-324 | Mature 5' sub | 21 | 19 | 58 | -2.09 | 0.821 | 5.34 |
| Example 245 | 245 | tRF | Homo sapiens tRNA-Phe-GAA-1-1//...*35 | Exact | 25 | 19 | 4 | -2.11 | 0.804 | 4.18 |
| Example 246 | 246 | tRF | Homo sapiens tRNA-Ser-GCT-1-1//...*32 | Exact | 23 | 12 | 118 | -2.13 | 0.797 | 3.78 |
| Example 247 | 247 | (RF | Homo sapiens tRNA-Val-AAC-1-1//...*5 | Exact | , 28 | 11 | 52 | -2.17 | 0.870 | 4.3 1 |
| Example 248 | 248 | tRF | Homo sapiens tRNA-Lys-CTT-1-1//...*10 | Exact | 26 | 10 | 39 | -2.21 | 0.851 | 4.37 |
| Example 249 | 249 | tRF | Homo sapiens tRNA-Lys-CTT-1-1//...*7 | Exact | 25 | 17 | 73 | -2.21 | 0.879 | 5.00 |
| Example 250 | 250 | tRF | Homo sapiens tRNA-Val-AAC-1-1//...*5 | Exact | 30 | 18 | 109 | -2.21 | 0.776 | 4.90 |
| Example 251 | 251 | tRF | Homo sapiens tRNA-Val-AAC-1-1//...*5 | Exact | 23 | 330 | 1721 | -2.23 | 0.894 | 9.67 |
| Example 252 | 252 | tRF | Homo sapiens tRNA-Glu-CTC-1-1//...*3 | Exact | 25 | 17 | 66 | -2.27 | 0.857 | 4.90 |
| Example 253 | 253 | tRF | Homo sapiens tRNA-Glu-CTC-1-1//...*3 | Exact | 29 | 25 | 148 | -2.33 | 0.835 | 5.69 |
| Example 254 | 254 | tRF | Homo sapiens tRNA-Ala-AGC-8-1//...*27 | Exact | 25 | 57 | 302 | -2.34 | 0.878 | 6.90 |
| Example 255 | 255 | tRF | Homo sapiens tRNA-Val-TAC-1-1//...*28 | Exact | 26 | 10 | 34 | -2.41 | 0.844 | 3.78 |
| Example 256 | 256 | tRF | Homo sapiens tRNA-Cys-GCA-2-1//...*30 | Exact | 31 | 8 | 47 | -2.44 | 0.808 | 2.92 |
| Example 257 | 257 | tRF | Homo sapiens tRNA-Ala-AGC-8-1//...*27 | Exact | 24 | 14 | 63 | -2.50 | 0.865 | 4.96 |
| Example 258 | 258 | tRF | Homo sapiens tRNA-Trp-CCA-3-1//...*29 | Exact | 24 | 20 | 119 | -2.53 | 0.846 | 5.59 |
| Example 259 | 259 | tRF | Homo sapiens tRNA-Val-AAC-1-1//...*5 | Exact | 26 | 29 | 160 | -2.62 | 0.885 | 6.06 |
| Example 260 | 260 | tRF | Homo sapiens tRNA-Val-AAC-1-1//...*5 | Exact | 24 | 15 | 83 | -2.63 | 0.876 | 4.73 |

**Table 2-11**

| Example | SEQ ID NO: | Class | Archetype | Type | Length (nucleotides) | Average in breast cancer patients | Average in healthy subjects | log FC | AUC | Cut-off value |
|---|---|---|---|---|---|---|---|---|---|---|
| Example 261 | 261 | tRF | Homo sapiens tRNA-Ala-AGC-11-1 | Exact | 25 | 3 | 24 | -2.67 | 0.883 | 3.43 |
| Example 262 | 262 | tRF | Homo sapiens tRNA-Val-AAC-1-1//...*5 | Exact | 29 | 13 | 96 | -2.69 | 0.848 | 4.79 |
| Example 263 | 263 | tRF | Homo sapiens tRNA-Trp-CCA-2-1 | Exact | 24 | 34 | 340 | -2.84 | 0.876 | 6.63 |
| Example 264 | 264 | tRF | Homo sapiens tRNA-Ala-AGC-8-1//...*27 | Exact | 24 | 3 | 22 | -2.92 | 0.920 | 2.45 |
| Example 265 | 265 | tRF | Homo sapiens tRNA-Val-AAC-1-1//...*5 | Exact | 2 5 | 9 | 66 | -3.03 | 0.890 | 4.62 |
| Example 266 | 266 | isomiR | mir-21 5p | Mature 5' super | 23 | 1778 | 799 | 1.15 | 0.785 | 9.97 |
| Example 267 | 267 | isomiR | mir-23a 3p | Mature 3' super | 22 | 2700 | 1403 | 0.94 | 0.785 | 10.51 |
| Example 268 | 268 | isomiR | mir-27a 3p | Mature 3' sub | 20 | 1119 | 436 | 1.36 | 0.85 | 8.93 |
| Example 269 | 269 | MiscRNA | ENST00000364600.1//...*36 | Exact | 28 | 1857 | 1227 | 0.60 | 0.744 | 10.11 |

| | | | | | | | | | | |
|---|---|---|---|---|---|---|---|---|---|---|
| *1 to *36 in this table represent the same molecules represented by *1 to *36 in Table 1. | | | | | | | | | | |

As seen in these results, the abundance of the miRNAs or the like represented by SEQ ID NOs: 1 to 19, 27, 28, 34 to 51, 74, 76, 77, 80 to 84, 96, 101 to 104, 115 to 122, 125, 128, 134 to 139, 151, 152, 159 to 165, 168, 169, 174, and 175 to 199 was significantly higher in the patients with breast cancer than in the healthy subjects, while the abundance of the miRNAs or the like represented by SEQ ID NOs: 20 to 26, 29 to 33, 52 to 54, 56 to 73, 75, 78 to 79, 85 to 95, 97 to 100, 105 to 114, 123, 124, 126, 127, 129 to 133, 140 to 150, 153 to 158, 166, 167, 170 to 173, and 200 to 265 was significantly lower in the patients with breast cancer than in the healthy subjects. It was indicated that breast cancer was able to be detected with higher accuracy by the method of the present invention (Examples 1 to 265), when compared with using, as indexes, miRNAs or the like (Comparative Examples 1 to 13) that are slightly different in length from those used in the method of the present invention. Moreover, most of the p-values determined by *t*-test in Examples 1 to 265 were less than 0.05, indicating the effectiveness in detection of breast cancer.

Moreover, stage 0 breast cancer was also able to be detected by the methods in which those represented by SEQ ID NOs: 3 to 9 were used as indexes. Furthermore, those represented by SEQ ID NOs: 2, 21, 22, 23, 24, 26, 30, 31, 32, 33, 34. 152, 151, 15, 28, 41, 1.14, 27, 40, 25, 12, and 160 have an AUC value of 0.97 or higher and are especially preferable. Furthermore, it was indicated that the abundance of the miRNAs or the like represented by SEQ ID NOs: 2, 21, 22, 23, 24, 26, 31 to 33. and 55 was zero in either cancer patients or healthy subjects, and use of those miRNAs or the like thus enabled high accuracy detection, similarly to use of miRNAs or the like having an AUC value of 1.00.

### Example 270

Similarly to Examples 1 to 269, the abundance of miR-150-5p (SEQ ID NO: 83) and miR-26b-5p (SEQ ID NO: 126) in the mature miRNA form ("mature" in Table 3) and the abundance of isoforms (isomiRs) of each of the miRNAs contained in serum were measured. In this respect, "the abundance of isoforms (isomiRs)" refers to the total abundance of sequences in which 1 to 5 nucleotides are deleted from or added to the 3' or 5' end of a mature miRNA. The abundance ratio between each miRNA and isoforms thereof was measured. The results are shown in Table 3 below.

### Example 271

Similarly to Examples 1 to 269, the abundance of miR-93-5p (SEQ ID NO: 155) and/or miR-17-5p (SEQ ID NO: 282) in the mature miRNA form ("mature" in Table 3) and the abundance of isoforms (isomiRs) of each of the miRNAs contained in serum were measured. In this respect, "the abundance of isoforms (isomiRs)" refers to the total abundance of sequences in which 1 to 5 nucleotides are deleted from or added to the 3' or 5' end of a mature miRNA. The abundance ratio between each miRNA and isoforms thereof was measured. The results are shown in Table 3 below.

**Table 3**

| | Ratio (isomiR/mature) | | (Breast cancer/Healthy subjects) | | | | |
|---|---|---|---|---|---|---|---|
| | Average in breast cancer patients | Average in healthy subjects | AUC | Cut-off value | Fold Change | p-value | In breast cancer |
| miR-150-5p | 0.62 | 0.37 | 0.899 | 1.68 | 1.68 | 2.13E-17 | isomiR > mature |
| miR-26b-5p | 0.73 | 0.37 | 0.816 | 1.98 | 1.98 | 9.73E-08 | isomiR > mature |
| miR-93-5p | 0.18 | 0.29 | 0.796 | 0.60 | 0.60 | 2.23E-08 | isomiR < mature |
| miR-17-5p | 2.26 | 3.18 | 0.783 | 0.71 | 0.71 | 7.07E-05 | isomiR < mature |

As indicated in Table 3, a higher isomiR/mature miRNA ratio than that of healthy subjects in the measurement of miR-150-5p (SEQ ID NO: 83) and miR-26b-5p (SEQ ID NO: 126) indicated a higher likelihood of having breast cancer, while a lower isomiR/mature miRNA ratio than that of healthy subjects in the measurement of miR-93-5p (SEQ ID NO: 155) and miR-17-5p (SEQ ID NO: 282) indicated a higher likelihood of having breast cancer.

## Claims

1. A method of assisting the detection of breast cancer, using as an index the abundance of at least one of miRNAs, isoform miRNAs (isomiRs), precursor miRNAs, transfer RNA fragments (tRFs), or non-coding RNA fragments (RRNAs, snoRNAs, or LincRNAs) contained in a test sample isolated from a living body, whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, 160, 3 to 11, 13, 16 to 20, 29, 35 to 39, 42 to 150, 153 to 159, and 161 to 269, wherein a higher abundance of at least one of the miRNAs, isomiRs, precursor miRNAs, transfer RNA fragments, or non-coding RNA fragments whose nucleotide sequence is represented by any one of SEQ ID NOs: 1 to 19, 27, 28, 34 to 51, 74, 76, 77, 80 to 84, 96, 101 to 104, 115 to 122, 125, 128, 134 to 139, 151, 152, 159 to 165, 168, 169, 174, and 175 to 199 than that of healthy subjects or a lower abundance of at least one of the miRNAs, isomiRs, precursor miRNAs, transfer RNA fragments, or non-coding RNA fragments whose nucleotide sequence is represented by any one of SEQ ID NOs: 20 to 26, 29 to 33, 52 to 54, 56 to 73, 75, 78 to 79, 85 to 95, 97 to 100, 105 to 114, 123, 124, 126, 127, 129 to 133, 140 to 150, 153 to 158, 166, 167, 170 to 173, and 200 to 269 than that of healthy subjects indicates a higher likelihood of having breast cancer.

2. The method according to claim 1, wherein the abundance of at least one of miRNAs, isoform miRNAs (isomiRs), precursor miRNAs, or transfer RNA fragments (tRFs) whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, 160, 3 to 11, 13, 16 to 20, 29, 35 to 39, 42 to 150, 153 to 159, and 161 to 174 is used as an index.

3. The method according to claim 1, wherein the abundance of at least one of miRNAs, isomiRs, precursor miRNAs, transfer RNA fragments, or non-coding RNA fragments whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, 160, 3 to 11, 13, 16, 20, 27, 29, 37 to 39, 41, 43, 45, 47 to 52, 56, 60, 66, 82, 86, 90 to 92, 107, 111, 112, 126, 127, 130, 137, 158, 161, 162, 173, and 175 to 265 is used as an index.

4. The method according to claim 3, wherein the abundance of at least one of miRNAs, isomiRs, precursor miRNAs, or transfer RNA fragments whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, 160, 3 to 11, 13, 16, 20, 27, 29, 37 to 39, 41, 43, 45, 47 to 52, 56, 60, 66, 82, 86, 90 to 92, 107, 111, 112, 126, 127, 130, 137, 158, 161, 162, and 173 is used as an index.

5. The method according to claim 4, wherein the abundance of at least one of isomiRs or precursor miRNAs whose nucleotide sequence is represented by any one of SEQ ID NOs: 3 to 9 is used as an index.

6. The method according to claim 2, wherein the abundance of at least one of miRNAs, isomiRs, precursor miRNAs, or transfer RNA fragments whose nucleotide sequence is represented by any one of SEQ ID NOs: 2, 21, 22, 23, 24, 26, 30, 31, 32, 33, 34, 152, 151, 15, 28, 41, 1, 14, 27, 40, 25, 12, and 160 is used as an index.

7. The method according to claim 6, wherein the abundance of at least one of isomiRs or transfer RNA fragments whose nucleotide sequence is represented by SEQ ID NO: 152, 151, 15, 40, 41, 1, or 14 is used as an index.

8. The method according to claim 2, wherein the abundance of at least one of isomiRs, precursor miRNAs, or transfer RNA fragments whose nucleotide sequence is represented by any one of SEQ ID NOs: 2, 21, 22, 23. 24, 26, 31 to 33, and 55 is used as an index.

9. The method according to claim 2, comprising measuring the abundance ratio of isoforms (isomiRs) of miR-150-5p (SEQ ID NO: 83) and/or miR-26b-5p (SEQ ID NO: 126) to the same microRNA(s) in the mature miRNA form contained in serum or plasma isolated from a living body (where "the abundance of isoforms (isomiRs)" refers to the total abundance of sequences in which 1 to 5 nucleotides are deleted from or added to the 3' or 5' end of a mature miRNA), wherein a higher abundance ratio than that of healthy subjects indicates a higher likelihood of having breast cancer.

10. The method according to claim 2, comprising measuring the abundance ratio of isoforms (isomiRs) of miR-93-5p (SEQ ID NO: 155) and/or miR-17-5p (SEQ ID NO: 282) to the same microRNA(s) in the mature miRNA form contained in serum or plasma isolated from a living body (where "the abundance of isoforms (isomiRs)" refers to the total abundance of sequences in which 1 to 5 nucleotides are deleted from or added to the 3' or 5' end of a mature miRNA), wherein a lower abundance ratio than that of healthy subjects indicates a higher likelihood of having breast cancer.
